# EUROPEAN PATENT APPLICATION

(11) **EP 4 477 736 A1**
(43) Date of publication of application: **18.12.2024**
(21) Application number: 24182150.3
(22) Date of filing: 14.06.2024
(51) Int. Cl.: C12N 1/00, C12N 15/11, C12N 15/63, C12N 15/67, C12N 15/70, C12N 15/77, C12P 21/02

(54) **GENETIC BIOCONTAINMENT SYSTEM TO PREVENT THEFT AND MISUSE OF BIOLOGICAL RESOURCES AND USES THEREOF**

(30) Priority: 14.06.2023 KR 20230076406
(71) Applicant: POSTECH Research and Business Development Foundation, Pohang-si, Gyeongsangbuk-do 37673 (KR)
(72) Inventor: LEE, Jeong Wook, 37673 Pohang-si, Gyeongsangbuk-do (KR); CHOI, Yun-nam, 37673 Pohang-si, Gyeongsangbuk-do (KR); KIM, Dong Hyeon, 37673 Pohang-si, Gyeongsangbuk-do (KR); LEE, Seong Beom, 37673 Pohang-si, Gyeongsangbuk-do (KR)
(74) Representative: Frenkel, Matthias Alexander

(57) **Abstract**

The present invention relates to a genetic biocontainment technology to prevent theft or misuse of biological resources (e.g., nucleic acid molecule, plasmid or microbial strain containing the nucleic acid molecule, or sequence information associated with the nucleic acid molecule) using a quadruplet codon and decoding system thereof. In particular, the present invention relates to a system or a microbial strain comprising a nucleic acid molecule in which a triplet codon encoding a specific amino acid in a nucleic acid sequence has been replaced with a quadruplet codon, wherein genetic biocontainment occurs in an environment where tRNA for the quadruplet codon does not exist, and a method using the same. The present invention also relates to a biocontainment method to protect sequence information using a quadruplet codon.

## Description

### CROSS-REFERENCE TO RELATED APPLICATIONS

The present application is based on, and claims priority from, Korean Patent Applications No. 10-2023-0076406 filed on June 14, 2023, the disclosure of which is hereby incorporated by reference herein in its entirety.

### TECHNICAL FIELD

The present invention relates to a genetic biocontainment technology to prevent theft or misuse of biological resources (e.g., nucleic acid molecule, plasmid or microbial strain containing the nucleic acid molecule, or sequence information associated with the nucleic acid molecule) using a quadruplet codon and decoding system thereof. In particular, the present invention relates to a system or a microbial strain comprising a nucleic acid molecule in which a triplet codon encoding a specific amino acid in a nucleic acid sequence has been replaced with a quadruplet codon, wherein genetic biocontainment occurs in an environment where tRNA for the quadruplet codon does not exist, and a method using the same. The present invention also relates to a biocontainment method to protect sequence information using a quadruplet codon.

This invention was made with support from the Samsung Future Technology Incubation Program (Project No.: SRFC-MA1901-11).

### BACKGROUND OF THE INVENTION

As a representative example of the conventional genetic biocontainment technology to prevent the theft or misuse of biological resources, there is a known technology that removes genes involved in the synthesis of essential amino acids or nucleic acids of a microbial strain, making it dependent on the corresponding components supplied from the culture environment. However, as the strain can survive when essential nutrients are supplied externally, it is easy to avoid the containment technique. In addition, there is a known technology to determine the survival of the microbial strain by the presence or absence of non-natural amino acids (ncAA) by including ncAA in essential enzymes, but there is a problem that requires significant genome modifications and an environmental problem caused by the use of non-natural amino acids. Furthermore, there is a known technology for activating the death pathway in the absence of survival signals in normal conditions, but there is a problem with the relatively high incidence of escaped cells. Moreover, there is a known technology that uses the CRISPR enzyme to delete core genetic information when a specific disruption signal is received, but there is a problem in that it is impossible to control microbial strains, which can lead to escape cells.

Therefore, there is a need for the development of technology that can efficiently achieve biocontainment while minimizing engineering of the microbial strain to be manipulated.

### SUMMARY OF THE INVENTION

The present invention relates to a genetic biocontainment technology to prevent theft or misuse of biological resources (e.g., nucleic acid molecule, plasmid or microbial strain containing the nucleic acid molecule, or sequence information associated with the nucleic acid molecule) using a quadruplet codon and decoding system thereof.

An embodiment described herein provides a composition for genetic biocontainment, comprising a nucleic acid molecule in which a triplet codon encoding a specific amino acid in a nucleic acid sequence has been replaced with a quadruplet codon, wherein the nucleic acid molecule can be expressed as a protein only in the presence of the quadruplet codon decoding system.

An embodiment described herein provides a genetic biocontainment system, comprising a nucleic acid molecule in which a triplet codon encoding a specific amino acid in a nucleic acid sequence has been replaced with a quadruplet codon, wherein the nucleic acid molecule can be expressed as a protein only in the presence of the quadruplet codon decoding system.

Another embodiment described herein provides a microbial strain for use in genetic biocontainment, comprising a nucleic acid molecule in which a triplet codon encoding a specific amino acid in a nucleic acid sequence has been replaced with a quadruplet codon, and a decoding system for the quadruplet codon, wherein the decoding system works under certain conditions.

Another embodiment described herein provides a genetic biocontainment method using the composition, the system or the microbial strain.

Another embodiment described herein provides a biocontainment method for sequence information comprising: replacing a triplet codon encoding a specific amino acid in a nucleic acid sequence with a quadruplet codon to cause a frameshift.

### BRIEF DESCRIPTION OF THE DRAWINGS

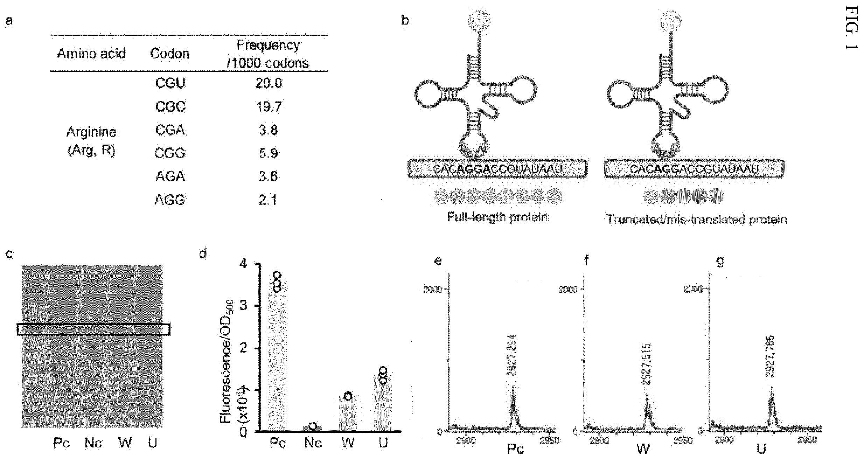

FIG. 1a shows the frequency of codons coding for arginine. FIG. 1b shows that the target gene containing the Q-codon (AGGA) is translated by Q-tRNA in which the anticodon is replaced with UCCU and the full-length protein is expressed, but the target gene containing the Q-codon is not properly translated by native N-tRNA in which the anticodon is not replaced. FIG. 1c and FIG. 1d show fluorescent measurement result (FIG. 1c) and SDS-PAGE result (FIG. 1d) confirming that Q-GFP was successfully expressed using two types of Q-tRNA (i.e., Q-tRNAw (W) and QtRNAu (U)). FIGs. 1e-g show the results of confirming the incorporation of arginine at the Q-codon site by MALDI-TOF analysis (Pc represents positive control, W represents Q-tRNAw, and U represents QtRNAu).

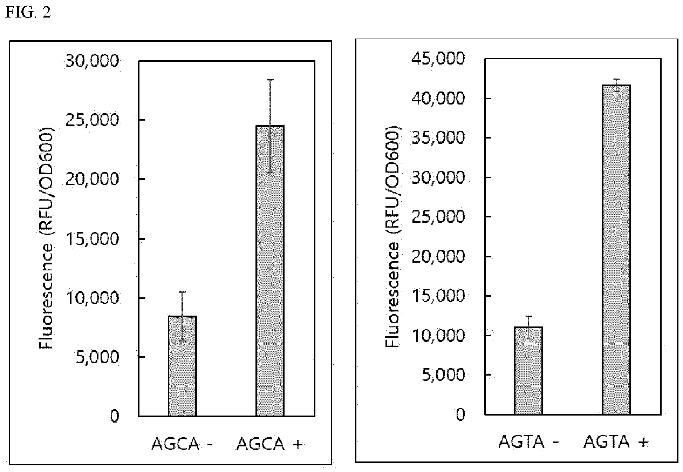

FIG. 2 shows fluorescent measurement results confirming that Q-GFP was translated and successfully expressed by Q-tRNA in which the anticodon was replaced with UGCU and UACU in each expression system constructed using the Q-codons AGCA and AGTA (-represents the expression of GFP containing a Q-codon in the absence of Q-tRNA and + represents the expression of GFP containing a Q-codon in the presence of Q-tRNA.).

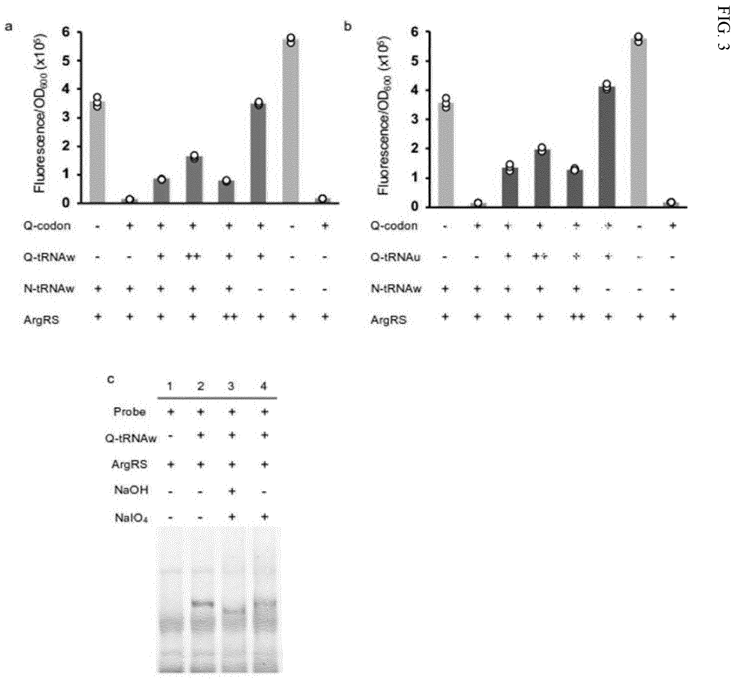

FIG. 3a shows fluorescent measurement results confirming the level of Q-GFP expression when Q-tRNAw was overexpressed or ArgRS was overexpressed. FIG. 3b shows fluorescent measurement results confirming the level of Q-GFP expression when Q-tRNAu was overexpressed or ArgRS was overexpressed. FIG. 3c shows the results of confirming the aminoacylation status of Q-tRNAw using tREX (tRNA Extension).

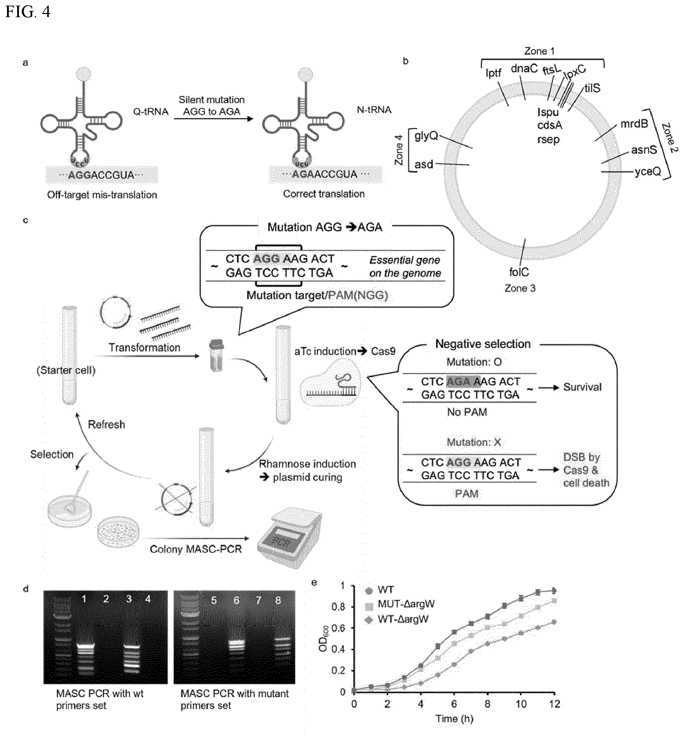

FIG. 4a shows that if AGGA is present because the base immediately following the triplet codon AGG happens to be A (off-target), which is not the Q-codon (AGGA), the four-base can be misread as a Q-codon by Q-tRNA, and this misreading can be prevented by silent mutation of AGG to AGA. FIG. 4b shows that 14 of the essential genes of *E. coli* contain AGGA within the CDS. FIG. 4c shows the process of replacing AGGA within the 14 CDS by AGAA using Cos-CRMAGE. FIG. 4d shows SDS-PAGE results confirming 14 mutations (MUTs). FIG. 4e shows the results confirming that cell growth was restored after introducing Q-tRNAu into a strain with 14 mutations (MUT).

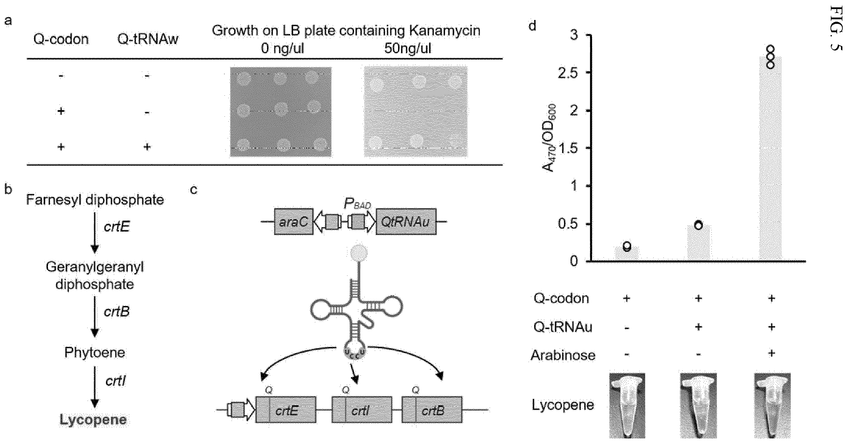

FIG. 5a shows the results confirming that cells containing Q-tRNAw can survive in response to kanamycin when a Q-codon is introduced into the kanamycin resistance gene. FIG. 5b shows lycopene production pathway. FIG. 5c shows the outline of an experiment in which a Q-codon is introduced into lycopene production pathway genes and Q-tRNAw is expressed under an inducible promoter. FIG. 5d shows the results confirming that lycopene is produced only when Q-tRNA is co-expressed when introducing a Q-codon into the lycopene production pathway genes.

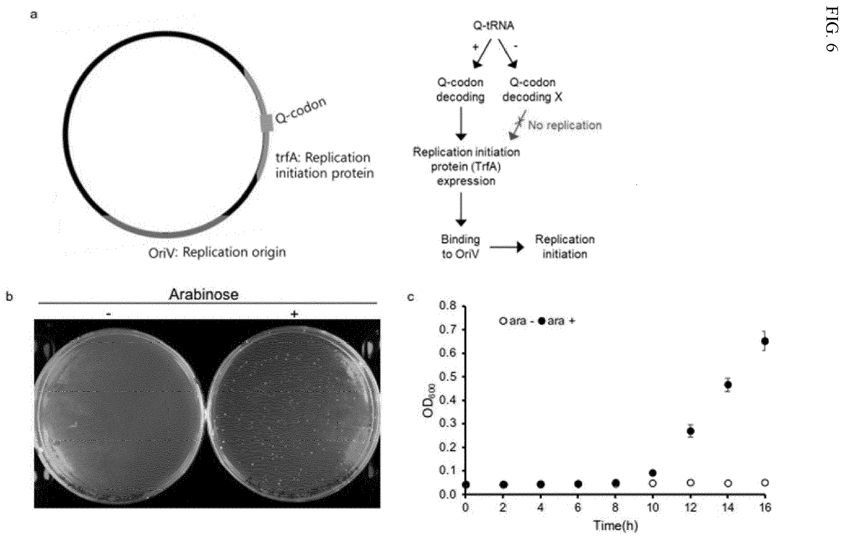

FIG. 6a shows a schematic diagram of a plasmid containing the replication initiation protein *TrfA* in which a Q-codon is introduced and the replication origin oriV, and experimental outline. FIG. 6b and FIG. 6c show results confirming that the plasmid is maintained only when Q-tRNAw is expressed, using Q-tRNAw under the control of an inducible promoter operated by arabinose.

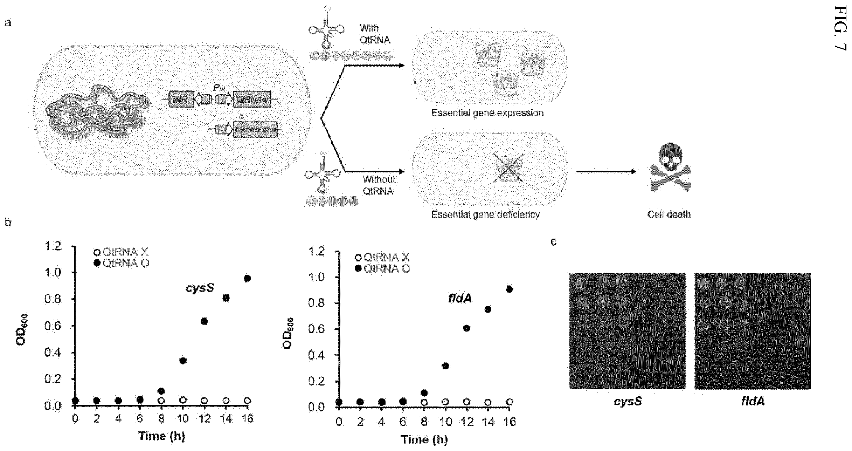

FIG. 7a shows an experimental outline for inserting a Q-codon into an essential enzyme responsible for cell growth and expressing Q-tRNAw under the control of an inducible promoter to prevent leakage of the engineered strain. FIG. 7b and FIG. 7c show the results confirming that when Q-codons are inserted into essential genes (cycS, fldA), cells can survive only in an environment where QtRNA is expressed.

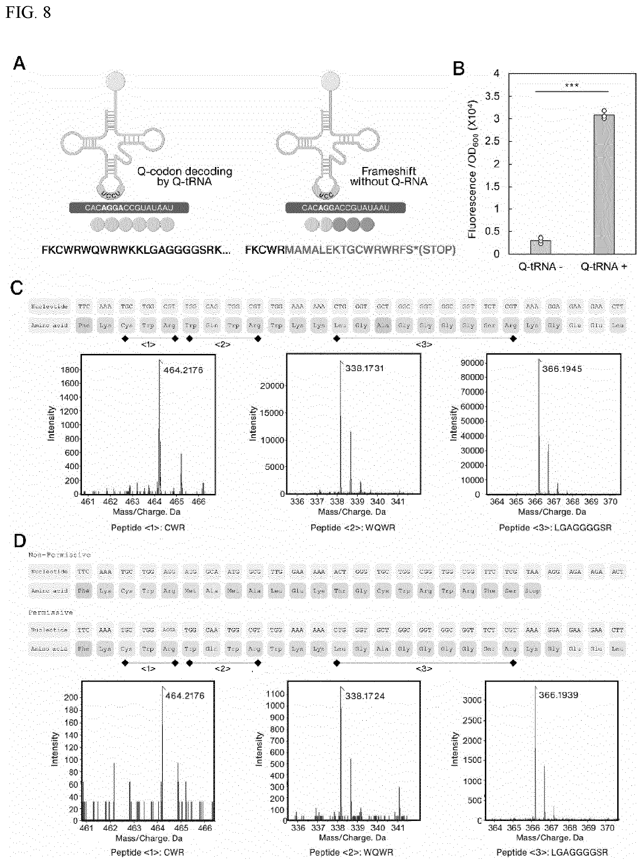

FIG. 8a indicates translation of the lactoferricin B with and without engineered tRNA capable of reading the quadruplet codon (AGGA). FIG. 8b indicates translation of lactoferricin B-GFP fusion protein based on the Q-codon decoding system confirmed by GFP fluorescence measurement (n=3, error bars indicate ±SD). Unpaired t tests were used for comparisons. ***P < 0.001. FIG. 8c and FIG.8d indicate correct translation in the presence of Q-tRNA confirmed using MALDI-TOF analysis. Peptides resulting from fragmentation around the arginine (R) encoded by the Q-codon exhibit the same mass as the positive control, not containing the Q-codon in the lactoferricin B sequence.

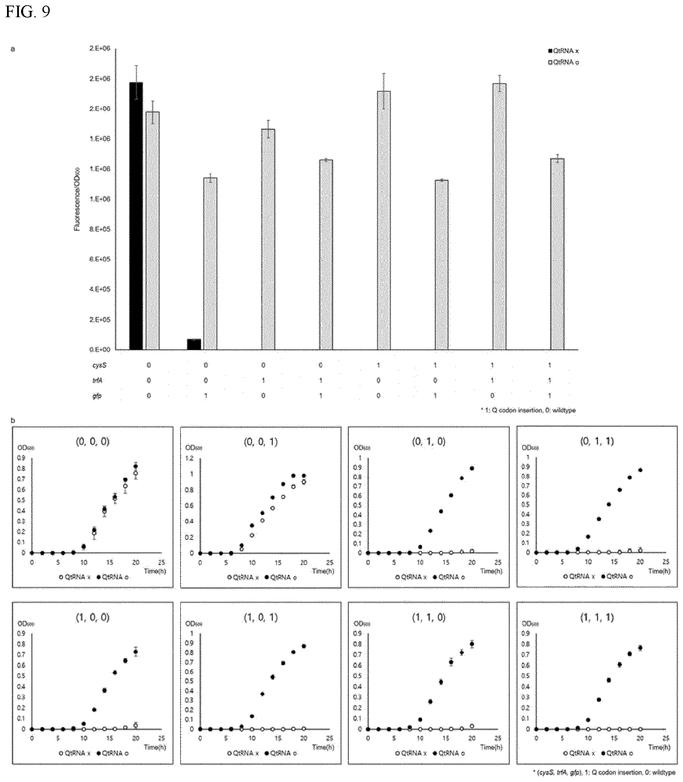

FIG. 9 shows the results of confirming the survival of strains in each combination when the Q codon is present or absent in the essential gene (*cysS*)*,* plasmid replication initiation gene (*trfA*), and target protein gene (*gfp*)*.* 0 in parentheses means that the Q codon does not exist, and 1 means that the Q codon exists. The genes corresponding to each position represent *cysS, trfA,* and *gfp* in that order.

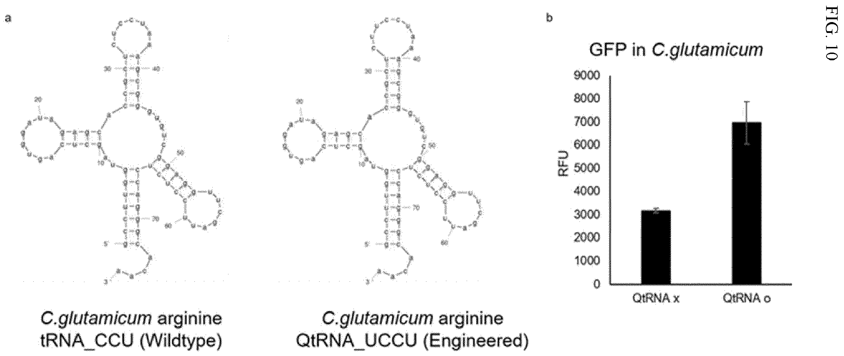

FIG. 10a shows improved tRNA (QtRNA) to construct the Q-codon system in *Corynebacterium glutamicum.* FIG. 10b shows the results confirming that GFP is expressed in the presence of QtRNA when GFP containing a Q-codon is introduced into *Corynebacterium glutamicum.*

### DETAILED DESCRIPTION OF THE INVENTION

A quadruplet codon (hereinafter also referred to as a 'Q-codon') is a codon composed of four nucleotides, unlike the existing triplet codon. It was mainly used as an expanded codon to incorporate new non-canonical amino acids into peptides or proteins in addition to the 20 naturally occurring amino acids. However, in order to use the Q-codon as an expanded codon, it is essential to introduce a tRNA/aminoacyl-tRNA synthetase (AARS) orthogonal pair derived from another strain that does not interfere with the strain's native tRNA and AARS. Thus, there is a problem in that the introduction of such an orthogonal pair may act as a burden to the strain.

However, the present invention is characterized by applying the Q-codon and its decoding system to a new field called genetic biocontainment. In the present invention, some of the existing triplet codons encoding natural amino acids in the sequence of nucleic acid molecules are replaced with Q-codons and a system for decoding the Q-codon is constructed. In addition, as the nucleic acid molecule containing the Q-codon can be expressed into a protein only when the Q-codon decoding system exists or operates, genetic biocontainment to prevent theft or misuse of biological resources (e.g., nucleic acid molecule, plasmid or microbial strain containing the nucleic acid molecule, or sequence information associated with the nucleic acid molecule) can be achieved. Furthermore, in the present invention, unlike the case of using the Q-codon as an expanded codon, the strain's endogenous tRNA/AARS can be used with some modification rather than using an orthogonal pair of tRNA/AARS derived from another strain, and thus, a burden to the strain is minimized and the present invention can be easily applied to various strains.

Specifically, in one embodiment of the invention, considering that three of the four nucleotides of the Q-codon necessarily overlap with existing triplet codons, we used rare codons to minimize interference, and selected AGG, the rarest codon encoding arginine, and its tRNA as the manipulation target. Thus, Q-codons (e.g., AGGA, AGCA or AGTA) created based on AGG, a rare codon for arginine, were used, and the anticodon of the endogenous tRNA, which decodes the original triplet codon AGG, was changed to UCCU, UGCU or UACU (Q-tRNA) to utilize the strain's unique system with minimal engineering. To test Q-codon decoding by the Q-tRNAs, the arginine codon of green fluorescent protein (GFP) was substituted with Q-codon (Q-GFP) and expressed together with Q-tRNA. As a result, green fluorescence was observed, full-length protein synthesis was confirmed through SDS-PAGE, and accurate incorporation of arginine at the Q-codon site was confirmed through MALDI-TOF analysis (FIG. 1, FIG. 2). On the other hand, full-length translation was not detected in the negative control strain without Q-tRNA. These results demonstrated that the Q-tRNAs were correctly charged with arginine and decoded the Q-codon in the Q-GFP.

Furthermore, in one example of the present invention, the Q-codon decoding efficiency was greatly improved by using a strain (WT-*ΔargW* strain) in which the strain's endogenous tRNA gene, *argW* that is in competition with the Q-tRNAw of the present invention, was knocked out in the genome (FIG. 3).

In addition, in a similar manner as above, the anticodon of another endogenous tRNA for arginine (argU) was changed to UCCU (Q-tRNAu), and the Q-codon decoding effect was confirmed in GFP (Q-GFP) into which the Q-codon AGGA was also introduced. However, since cell growth was observed to be significantly reduced in the *ΔargW* strain expressing Q-tRNAu, additional genome engineering was performed to reduce the off-target effect. Specifically, among the genes for enzymes essential for cell growth, it was observed that AGGA, which can cause off-target effects, exists in a total of 14 regions, and thus, we changed AGGA to AGAA in these refions (*MUT-ΔargW* strain). As a result, it was confirmed that cell growth was restored (FIG. 4).

Thus, it was possible to construct a genetic biocontainment system to protect a genetic trait such as a target gene or metabolic synthesis pathway gene (FIG. 5), genetic material such as a plasmid (FIG. 6), or engineered strain (FIG. 6) and construct an integrated biocontainment system by modifying the sequence of the nucleic acid molecule using the Q-codon for arginine, and by using Q-tRNAw as a decoding system in the wild type (WT) or wild-type *ΔargW* strain (WT*-ΔargW*) or by using Q-tRNAu as a decoding system in the mutant *ΔargW* strain (MUT-*ΔargW*)*.* Furthermore, it was possible to construct a biocontainment system using "intentional frameshift" by inserting Q-codon as a means of sequence encryption (Table 9).

Thus, according to one aspect of the present invention, there is provided a composition for genetic biocontainment, comprising a nucleic acid molecule in which a triplet codon encoding a specific amino acid in a nucleic acid sequence has been replaced with a quadruplet codon, wherein the nucleic acid molecule is expressed as a protein only in the presence of (i) a tRNA with an anticodon complementary to the quadruplet codon and (ii) an aminoacyl-tRNA synthetase (AARS) that recognizes and acylates the tRNA. Thus, the nucleic acid molecule is not expressed as a protein in the absence of the tRNA with an anticodon complementary to the quadruplet codon.

According to another aspect of the present invention, there is provided a genetic biocontainment system, comprising a nucleic acid molecule in which a triplet codon encoding a specific amino acid in a nucleic acid sequence has been replaced with a quadruplet codon, wherein the nucleic acid molecule is expressed as a protein in the presence of (i) a tRNA with an anticodon complementary to the quadruplet codon and (ii) an aminoacyl-tRNA synthetase (AARS) that recognizes and acylates the tRNA. Thus, the nucleic acid molecule is not expressed as a protein in the absence of the tRNA with an anticodon complementary to the quadruplet codon.

According to another aspect of the present invention, there is provided a microbial strain for use in genetic biocontainment, comprising a nucleic acid molecule in which a triplet codon encoding a specific amino acid in a nucleic acid sequence has been replaced with a quadruplet codon, wherein the strain comprises quadruplet codon decoding system comprising (i) a tRNA with an anticodon complementary to the quadruplet codon and (ii) an aminoacyl-tRNA synthetase (AARS) that recognizes and acylates the tRNA, and wherein the expression of the tRNA is controlled under an inducible promoter, such that in the presence of an inducer of the inducible promoter, the tRNA is expressed and the nucleic acid molecule is expressed as a protein, and in the absence of the inducer, the tRNA is not expressed and the nucleic acid molecule is not expressed as a protein.

According to another aspect of the present invention, there is provided a genetic biocontainment method comprising: providing the composition or system as described above; and inducing or blocking the expression of a nucleic acid molecule containing the quadruplet codon depending on the presence or expression of a tRNA with an anticodon complementary to the quadruplet codon.

According to another aspect of the present invention, there is provided a biocontainment method for sequence information comprising: replacing a triplet codon encoding a specific amino acid in a nucleic acid sequence with a quadruplet codon to cause a frameshift.

Hereinafter, the present invention will be described in more detail.

Where the terms "comprise", "comprises", "comprised" or "comprising" are used in this specification (including the claims) they are to be interpreted as specifying the presence of the stated features, integers, steps or components, but not precluding the presence of one or more other feature, integer, step, component or group thereof.

The discussion of documents, acts, materials, devices, articles and the like is included in this specification solely for the purpose of providing a context for the present invention. It is not suggested or represented that any or all of these matters formed part of the prior art base or were common general knowledge in the field relevant to the present invention before the priority date of each claim of this application.

According to one aspect of the present invention, there is provided a composition for genetic biocontainment, comprising a nucleic acid molecule in which a triplet codon encoding a specific amino acid in a nucleic acid sequence has been replaced with a quadruplet codon,
wherein the nucleic acid molecule is expressed as a protein only in the presence of (i) a tRNA with an anticodon complementary to the quadruplet codon and (ii) an aminoacyl-tRNA synthetase (AARS) that recognizes and acylates the tRNA, and the nucleic acid molecule is not expressed as a protein in the absence of the tRNA with an anticodon complementary to the quadruplet.

According to one aspect of the present invention, there is provided a genetic biocontainment system, comprising a nucleic acid molecule in which a triplet codon encoding a specific amino acid in a nucleic acid sequence has been replaced with a quadruplet codon,
wherein the nucleic acid molecule is expressed as a protein only in the presence of (i) a tRNA with an anticodon complementary to the quadruplet codon and (ii) an aminoacyl-tRNA synthetase (AARS) that recognizes and acylates the tRNA, and the nucleic acid molecule is not expressed as a protein in the absence of the tRNA with an anticodon complementary to the quadruplet.

The term "biocontainment" as used herein refers to preventing biological resources such as genetically modified nucleic acid molecules, plasmids, microbial strains, and sequence information from being leaked, thieved, stolen, or misused. Biocontainment may be necessary to prevent genetically modified biological resources from migrating to the external environment (e.g., nature) or other microorganisms, causing potential environmental or health risks. On the other hand, biocontainment may be necessary to prevent various biological resources that require a long period of research and development from being leaked or stolen.

The term "quadruplet codon" or "Q-codon" refers to a codon consisting of four nucleotides. Although theoretically there can be 256 combinations of quadruplet codons, considering that any Q-codon includes unavoidably one of the existing triplet codons, in order to minimize interference, it may be desirable to use rare codons that are less frequently used.

As used herein, the terms "nucleic acid molecule", "nucleic acid" and "polynucleotide" are used interchangeably and refer to a polymeric form of nucleotides of any length, either ribonucleotides or deoxyribonucleotides. Thus, nucleic acid molecule includes, but is not limited to, single-, double-, or multi- stranded DNA or RNA, genomic DNA, cDNA, DNA-RNA hybrids, or a polymer comprising purine and pyrimidine bases or other natural, chemically or biochemically modified, non-natural, or derivatized nucleotide bases. The nucleic acid of the invention can be prepared by any method known to one skilled in the art, including chemical synthesis, recombination, and mutagenesis. In preferred embodiments, the nucleic acid of the invention is a DNA molecule, preferably double stranded DNA molecule, which can be synthesized by recombinant methods well known to those skilled in the art.

In one embodiment, the nucleic acid molecule may be one, two or three types of nucleic acid molecules selected from the group consisting of a gene encoding a target protein, a gene encoding a plasmid replication initiation protein, and a gene encoding a protein essential for cell growth.

The term "a gene encoding a target protein" or "a target gene" as used herein refers to a gene encoding a protein to be introduced into a microbial strain from outside, and may be a polynucleotide sequence or an exogenous nucleic acid sequence encoding a target product (RNA or protein, etc.) to be expressed in a microbial strain. A target gene may include genes encoding exogenous proteins, endogenous proteins, or reporter proteins, and may encode proteins in native or mutant forms, but are not limited thereto. An exogenous protein refers to a protein that does not naturally exist in a specific tissue or cell, and an endogenous protein refers to a protein expressed by a gene that naturally exists in a specific tissue or cell. A reporter protein refers to a marker protein expressed by a reporter gene and can be used to quantify or detect its expression or activity in a cell by its presence. The sequence of a target gene may be in a truncated form, a fused form, or a tagged form, and may be cDNA or gDNA, but is not limited thereto.

In a preferred embodiment, a target gene may be a gene that imparts a specific phenotype or trait to the microbial strain, such as antibiotic-resistance genes that confer resistance to antibiotics (e.g., tetracycline, ampicillin, kanamycin, neomycin, hygromycin, bleomycin, methotrexate, or spectinomycin, etc.) or reporter genes that provide phenotypic changes or enzymatic properties (e.g., genes coding for glucuronidase, luciferase, β-galactosidase, β-lactamase, chloramphenicol acetyltransferase (CAT), fluorescent proteins (e.g., green fluorescent protein (GFP), yellow fluorescent protein (YFP)), etc.), but is not limited thereto. Furthermore, the target gene may be one or more genes involved in a metabolite synthesis pathway, but is not limited thereto.

The term "a gene encoding a plasmid replication initiation protein" as used herein refers to a gene encoding a protein that recognizes the origin of replication, which is the replication initiation site of the plasmid, and initiates plasmid DNA replication. Examples include, but are not limited to, TrfA, RepA, RepB, RepC, RepD, π, etc.

The term "a gene encoding a protein essential for cell growth" as used herein refers to a gene encoding a protein that can lead to growth inhibition and/or death of the microbial strain if not properly expressed, such as proteins essential for the survival of the microbial strain to be used, or proteins involved in the production of metabolites essential for survival.

In one embodiment, the nucleic acid molecule into which the Q-codon is introduced may be a gene encoding a target protein, wherein the nucleic acid molecule is not expressed as the target protein in the absence of the tRNA with an anticodon complementary to the quadruplet, thereby capable of achieving genetic biocontainment of the gene.

In another embodiment, the nucleic acid molecule into which the Q-codon is introduced may be a gene involved in a metabolite synthesis pathway, wherein metabolites are not synthesized in the absence of the tRNA with an anticodon complementary to the quadruplet, thereby capable of achieving genetic biocontainment of metabolite synthesis.

In another embodiment, the nucleic acid molecule into which the Q-codon is introduced may be a gene encoding a plasmid replication initiation protein, wherein the plasmid containing the nucleic acid molecule does not replicate in the absence of the tRNA with an anticodon complementary to the quadruplet, thereby capable of achieving genetic biocontainment of the plasmid.

In another embodiment, the nucleic acid molecule into which the Q-codon is introduced may be a gene encoding a protein essential for cell growth, wherein cell death does not occur in the absence of the tRNA with an anticodon complementary to the quadruplet, thereby capable of achieving genetic biocontainment of the microbial strain.

In a preferred embodiment, the nucleic acid molecule may be a nucleic acid molecule in which a triplet codon encoding arginine in a nucleic acid sequence has been replaced with a quadruplet codon. For example, the nucleic acid molecule may be a nucleic acid molecule in which a triplet codon encoding arginine in a nucleic acid sequence has been replaced with a quadruplet codon consisting of AGNN (N is A, T, C or G). Alternatively, the nucleic acid molecule may be a nucleic acid molecule in which a triplet codon encoding arginine in a nucleic acid sequence has been replaced with a quadruplet codon consisting of AGGN (N is A, T, C or G).

In another preferred embodiment, the nucleic acid molecule may be a nucleic acid molecule in which a triplet codon encoding arginine in a nucleic acid sequence has been replaced with a quadruplet codon consisting of AGGA, AGCA or AGTA. In this case, the tRNA with an anticodon complementary to the quadruplet codon may be a tRNA prepared by replacing the anticodon with UCCU, UGCU, or UACU, respectively, in the strain's endogenous tRNA for arginine.

In one aspect, the present invention provides a microbial strain for use in genetic biocontainment, comprising a nucleic acid molecule in which a triplet codon encoding a specific amino acid in a nucleic acid sequence has been replaced with a quadruplet codon. The features of biocontainment, quadruplet codon, and nucleic acid molecule are as described above.

The microbial strain herein is characterized in that it comprises quadruplet codon decoding system comprising (i) a tRNA with an anticodon complementary to the quadruplet codon and (ii) an aminoacyl-tRNA synthetase (AARS) that recognizes and acylates the tRNA, and wherein the expression of the tRNA is controlled under an inducible promoter, such that in the presence of an inducer of the inducible promoter, the tRNA is expressed and the nucleic acid molecule is expressed as a protein, and in the absence of the inducer, the tRNA is not expressed and the nucleic acid molecule is not expressed as a protein.

The term "inducible promoter" as used herein refers to a promoter whose activity can be increased or decreased upon an external stimulus. Stimuli can be physical or chemical in nature, such as temperature, light, chemicals (e.g., inducer) etc. In preferred embodiments, the inducible promoter may include, but are not limited to, P_{BAD}, which is a promoter of the L-arabinose operon (or araBAD operon); P_{Tet}, which is an anhydrotetracycline (ATc)-inducible promoter derived from the *E. coli* transposon Tn*10*; P_{tac}, which is a IPTG (isopropyl-β-D-1-thiogalactopyranoside)-inducible promoter, etc.

In a preferred embodiment, the nucleic acid molecule may be a nucleic acid molecule in which a triplet codon encoding arginine in a nucleic acid sequence has been replaced with a quadruplet codon. For example, the nucleic acid molecule may be a nucleic acid molecule in which a triplet codon encoding arginine in a nucleic acid sequence has been replaced with a quadruplet codon consisting of AGNN (N is A, T, C or G). In another embodiment, the nucleic acid molecule may be a nucleic acid molecule in which a triplet codon encoding arginine in a nucleic acid sequence has been replaced with a quadruplet codon consisting of AGGN (N is A, T, C or G).

In another preferred embodiment, the nucleic acid molecule may be a nucleic acid molecule in which a triplet codon encoding arginine in a nucleic acid sequence has been replaced with a quadruplet codon consisting of AGGA, AGCA or AGTA. In this case, the tRNA with an anticodon complementary to the quadruplet codon may be a tRNA prepared by replacing the anticodon with UCCU, UGCU, or UACU, respectively, in the strain's endogenous tRNA for arginine.

In the microbial strain herein, the tRNA with an anticodon complementary to the quadruplet codon and the aminoacyl-tRNA synthetase (AARS) that recognizes and acylates the tRNA may be a modified version of the endogenous tRNA/AARS system of the strain. For example, the tRNA/AARS system of the strain herein may be a modified version of the endogenous tRNA/AARS system for specific amino acids encoded by quadruplet codons, in which the anticodon of the endogenous tRNA is replaced with an anticodon complementary to the quadruplet codon and the endogenous AARS is modified to acylate the specific amino acid to tRNA. Therefore, in the present invention, unlike the prior art that uses the Q-codon as an expanded codon, the strain's endogenous tRNA/AARS can be used with some modification rather than using an orthogonal pair of tRNA/AARS derived from another strain, and thus, a burden to the strain is minimized and the present invention can be easily applied to various strains.

Preferably, the microbial strain may be additionally engineered to further improve Q-codon decoding efficiency using the tRNA.

In a preferred embodiment, the microbial strain herein may be additionally engineered to knock out the argW gene, which encodes the strain's endogenous tRNA for arginine. In this regard, in one example of the present invention, it was confirmed that the Q-codon decoding efficiency can be greatly improved by using a strain (WT-*ΔargW* strain) in which the strain's endogenous tRNA gene, *argW* that is in competition with the Q-tRNAw of the present invention, was knocked out in the genome (FIG. 3).

In another preferred embodiment, the microbial strain herein may be additionally engineered to replace AGNN with AGGA, AGCA or AGTA, for example, to replace AGGA bases with AGAA in the natural nucleic acid sequences of genes essential for cell growth in order to reduce off-target effects. In this regard, in one example of the present invention, it was confirmed that Q-codon decoding efficiency can be increased while maintaining cell growth by modifying a total of 14 AGGA sites among the genes for enzymes essential for cell growth to AGAA (FIG. 4).

In one embodiment, in the microbial strain herein, the nucleic acid molecule containing Q-codon may be a gene encoding a target protein. In this case, since the expression of the tRNA for the Q-codon is controlled under an inducible promoter, in the presence of the inducer, the tRNA is expressed and thus the nucleic acid molecule is expressed as a protein, but in the absence of the inducer, the tRNA is not expressed and thus the nucleic acid molecule is not expressed as a protein.

In another embodiment, the nucleic acid molecule containing Q-codon may be a gene involved in a metabolite synthesis pathway. In this case, since the expression of the tRNA for the Q-codon is controlled under an inducible promoter, in the presence of the inducer, the tRNA is expressed and thus metabolites are synthesized from the strain, but in the absence of the inducer, the tRNA is not expressed and thus metabolites are not synthesized from the strain.

In another embodiment, the nucleic acid molecule containing Q-codon may be a gene encoding a plasmid replication initiation protein. In this case, since the expression of the tRNA for the Q-codon is controlled under an inducible promoter, in the presence of the inducer, the tRNA is expressed and thus the plasmid containing the nucleic acid molecule is replicated from the strain, but in the absence of the inducer, the tRNA is not expressed and thus the plasmid containing the nucleic acid molecule is not replicated from the strain.

In another embodiment, the nucleic acid molecule containing Q-codon may be a gene encoding a protein essential for cell growth. In this case, since the expression of the tRNA for the Q-codon is controlled under an inducible promoter, in the presence of the inducer, the tRNA is expressed and thus the strain survives, but in the absence of the inducer, the tRNA is not expressed and thus the strain dies.

The microbial strain herein may be any strain known in the art that can clone or express a nucleic acid molecule into which a Q-codon has been introduced. For example, the strain may be a prokaryotic cell, which may include *E. coli,* such as *E. coli* JM109, *E. coli* BL21, *E. coli* RR1, *E. coli* LE392, *E. coli* B, *E. coli* X 1776, *E. coli* W3110; strains of the genus Bacillus, such as *Bacillus subtilis, Bacillus thuringensis;* strains of the genus Corynebacterium, such as *Corynebacterium glutamicum;* Enterobacteriaceae strains, such as *Salmonella Typhimurium, Serratia marcescens,* and various Pseudomonas species. Further, the microbial strain may be a eukaryotic cell, which may include yeast (*Saccharomyce cerevisiae*)*,* insect cells, plant cells, and animal cells, such as CHO cell lines (Chinese hamster ovary), W138, BHK, COS-7, 293, HepG2, 3T3, RIN, and MDCK cell lines, but is not limited thereto.

In another aspect, there is provided a genetic biocontainment method comprising: providing the composition, system or microbial strain as described above; and inducing or blocking the expression of a nucleic acid molecule containing the quadruplet codon depending on the presence or expression of a tRNA with an anticodon complementary to the quadruplet codon.

Such genetic biocontainment may be a genetic biological containment to protect 1) genetic traits, such as target genes or metabolic synthesis pathway genes, 2) genetic materials, such as plasmids, 3) engineered microbial strains (FIG. 6), or 4) genetic information, such as sequence information, but is not limited thereto.

Thus, in another aspect, there is provided a biocontainment method for sequence information comprising: replacing a triplet codon encoding a specific amino acid in a nucleic acid sequence with a quadruplet codon to cause a frameshift. If an "intentional frame shift" is caused by inserting a Q-codon instead of a triplet codon as a means of encoding sequence information, the nucleic acid sequence is changed, and thus protein identification becomes impossible because it is decoded and searched with a different amino acid sequence even using conventional programs that search sequences based on triplet codons, for example, BLAST (NCBI), CLC Main Workbench (CLC bio), MegAlign^{™} (DNASTAR Inc), etc. Thus, the biocontainment method herein can be further specified as comprising: replacing a triplet codon encoding a specific amino acid in a nucleic acid sequence with a quadruplet codon to cause a frameshift; searching for a nucleic acid sequence or amino acid sequence using conventional programs that search sequences based on triplet codons; and obtaining a nucleic sequence or amino acid sequence information that is different from the sequence before frameshift, whereby biocontainment can be achieved to prevent sequence information from being leaked, thieved, stolen, or misused.

### EXAMPLES

Hereinafter, the present invention will be described in more detail with reference to the following examples. However, these examples are only for illustrating the present invention, and the scope of the present invention is not limited by these examples.

### Example 1. Strains and cultivation

For cloning and plasmids construction, Escherichia coli (*E. coli*) DH10β was used, and for protein expression and quadruplet codon tests, *E. coli* DH10β and *E. coli* MG1655 were used. *E. coli* MG1655 is the parental strain for genome engineering, which includes *argW* gene knockout and mutation of AGGA to AGAA in the 14 essential genes.

For cell cultivation, a liquid or solid form of Luria-Bertani (LB) media were used with appropriated antibiotics (kanamycin: 50 ug/mL, carbenicillin: 100 ug/mL, gentamicin: 30 ug/mL, and chloramphenicol: up to 34 ug/mL, GoldBio). If not specified, cells were grown at 37°C, and agitation at 200 rpm was provided in case of liquid culture. Cells carrying temperature-sensitive plasmids were grown at 30°C. Cell growth was monitored by measuring optical density at 600 nm (OD₆₀₀) using a spectrophotometer (Biodrop) or multiplate reader (Hidex).

### Example 2. DNA manipulation

### 2-1. General Methodology

All the polymerase chain reactions (PCRs) were conducted using Q5 polymerase (NEB) according to the manufacturer's protocol, if not specified. All the oligonucleotides used in this study were purchased from Cosmogenetech, Korea. When necessary, PCR products were purified (Geneall PCR purification kit) after treatment with DpnI (NEB) for at least 3h at 37°C.

To amplify short DNA fragments ≤ 90-bp, oligo-dimerization was conducted by: 1) mixing two oligomers which are entirely complementary to each other, 2) heating them at 95°C for 2 min, and 3) slowly cooling them to room temperature for 1h.

To amplify short DNA fragments > 90-bp and ≤ 200-bp, polymerase cycling assembly (PCA) was conducted. Two seed oligonucleotides (up to 90-nt) which are complementary at their 3' ends in 20-nt length are hybridized, and each end is extended by polymerase reaction (18-20 cycles). Then, primers unique to the resulting ends are introduced into the reaction, and the target fragment is amplified by the regular PCR (30 cycles).

Plasmids were constructed by TA cloning (Promega), restriction enzyme digestion & ligation (NEB), or Gibson assembly (NEB) according to the manufacturer's protocol. After transformation, orientations and sequences were confirmed by colony PCR (Accupower PCR premix, Bioneer) and sequences analysis (Cosmogenetech).

Site-directed mutagenesis was performed by either a site-directed mutagenesis kit (NEB) or self-Gibson assembly. For both ways, plasmid is linearized by PCR, of which one primer is designed to include desired mutations. For the self-Gibson assembly, the primer with mutations is designed to also include at least 20-nt overhang that is complementary to the other primer, so that overlapping ends can be formed by PCR. The PCR products were treated by the enzyme mix provided in the kit or DpnI, purified, and transformed to the appropriate cells. The resulting mutation was confirmed by sequence analysis.

### 2-2. Plasmid Construction

To construct an expression plasmid for GFP with or without a quadruplet codon (Q-codon), pCDF-sfGFP (Addgene #) was used as a base plasmid after replacing its antibiotic resistance from ampicillin to kanamycin. Prior to the Q-codon replacement, one silent mutation at Leu-45 (CTG to CTT) was introduced by the self-Gibson assembly so that it can be read as a stop codon in case of frameshift, resulting in pGFP. Using the pGFP as a template plasmid, the Q-codon (AGGA) was inserted at the Arg-31 position in the sfGFP by the site-directed mutagenesis kit, resulting in pQGFP.

**[Table 1]**

| **Gene** | **Sequence** | **Mutation/premat ure termination point** |
|---|---|---|
| GFP | ATGGCTAGCAAGGGCGAGGAGCTGTTCACCGGGGTGG TGCCCATCCTGGTCGAGCTGGACGGCGACGTAAACGG CCACAAGTTCAGCGTGCGC¹GGCGAGGGCGAGGGCGA TGCCACCAACGGCAAGCTGACCCTG²AA³GTTCATCTG CACCACCGGCAAGCTGCCCGTGCCCTGGCCCACCCTCG TGACCACCCTGACCTACGGCGTGCAGTGCTTCAGCCGC TACCCCGACCACATGAAGCAGCACGACTTCTTCAAGTC CGCCATGCCCGAAGGCTACGTCCAGGAGCGCACCATC AGCTTCAAGGACGACGGCACCTACAAGACCCGCGCCG AGGTGAAGTTCGAGGGCGACACCCTGGTGAACCGCAT CGAGCTGAAGGGCATCGACTTCAAGGAGGACGGCAAC ATCCTGGGGCACAAGCTGGAGTACAACTTCAACAGCC ACAACGTCTATATCACCGCCGACAAGCAGAAGAACGG CATCAAGGCCAACTTCAAGATCCGCCACAACGTGGAG GACGGCAGCGTGCAGCTCGCCGACCACTACCAGCAGA ACACCCCCATCGGCGACGGCCCCGTGCTGCTGCCCGA CAACCACTACCTGAGCACCCAGTCCGTGCTGAGCAAA GACCCCAACGAGAAGCGCGATCACATGGTCCTGCTGG AGTTCGTGACCGCCGCCGGGATCACTCTCGGCATGGA CGAGCTGTACAAGTAA (SEQ ID NO: 1) | ¹ CGC to AGGA |
| | | ²G to T |
| | | ³ read as TAA(stop) after mutation |

To construct an expression cassette for Q-tRNAs that can suppress the Q-codon, AGGA, we first hybridized a pair of complementary oligonucleotides corresponding to a native tRNA for Arg encoded by argW or argU, respectively, except for its anticodon sequence, which was replaced by UCCU. We replaced tRNA_{Tyr} of pRF0G-Tyr, which was a gift from Jeffrey Barrick (Addgene plasmid #113644;http://n2t.net/addgene: 11364;RRID :Addgene _11364), with the product by Gibson assembly, yielding p15A_QtRNAw and p15A_QtRNAu, respectively. We then amplified the resulting expression cassette by PCR using primers set, FPLpp and RrrnBTT, which introduced BamHI and XhoI sites at each end, respectively. The PCR product was digested by BamHI and XhoI, and inserted downstream of the QGFP in the pQGFP, resulting in pQGFPw and pQGFPu, respectively. The same PCR products were ligated into pGEM-T-Easy vector (Promega) to construct a high-copy plasmid for Q-tRNA expression, yielding pH.QtRNAw and pH.QtRNAu, respectively. To construct arabinose-inducible Q-tRNA expression plasmids, a gene fragment containing *araC* and P_{BAD} was cloned by PCR using primers set, FaraC and Rprbad, and replaced a fragment containing the QGFP expression cassette and P_{LPP} in the pQGFPw and pQGFPu, resulting in pBADw and pBADu, respectively. A gene fragment including *araC* and the arabinose-inducible QtRNA expression cassette was cloned from pBADw and pBADu by PCR and ligated into pGEM-T-Easy, resulting in the high copy plasmids for the Q-tRNA expression, pH.Badw and pH.Badu, respectively. To construct an expression plasmid for kanamycin resistance gene with and without the Q-codon, we removed the expression cassettes for GFP from pGFP using PCR and self-Gibson assembly, yielding pKM and then we replaced the original arginine codon with AGGA by site-directed mutagenesis, yielding pQKM.

For lycopene production, we amplified a gene cluster including *crtE* encoding geranylgeranyl diphosphate synthase, *crtB* encoding phytoene synthase, and *crtI* encoding phytoene desaturase) from pAC-LCY, which was a gift from Francis X Cunningham Jr (Addgene plasmid # 53270; htttp://n2t.net/addgene:53270; RRID:Addgene_53270), by PCR using primers set, FcrtE and RcrtB. We replaced the GFP gene in the pGFP by the PCR product, resulting in pLYC. We inserted the Q-codon into one of the arginine sites located in *crtE, crtB,* and *crtI* (crtE:44R, crtB: 124R, crtI: 88R), yielding pQLYC.

**[Table 2]**

| **Gene** | **Sequence** | **Mutation/premat ure termination point** |
|---|---|---|
| CrtE | ATGGTGAGTGGCAGTAAAGCGGGCGTTTCGCCTCATC GCGAAATAGAAGTAATGAGACAATCCATTGACGATCA CCTGGCTGGCCTGTTACCTGAAACCGACAGCCAGGAT ATCGTCAGCCTTGCGATGCGT¹GAAGGCGTCATGGCAC CCGGTAA²ACGGATCCGTCCGCTGCTGATGCTGCTGGC CGCCCGCGACCTCCGCTACCAGGGCAGTATGCCTACG CTGCTCGATCTCGCCTGCGCCGTTGAACTGACCCATAC CGCGTCGCTGATGCTCGACGACATGCCCTGCATGGAC AACGCCGAGCTGCGCCGCGGTCAGCCCACTACCCACA AAAAATTTGGTGAGAGCGTGGCGATCCTTGCCTCCGTT GGGCTGCTCTCTAAAGCCTTTGGTCTGATCGCCGCCAC CGGCGATCTGCCGGGGGAGAGGCGTGCCCAGGCGGTC AACGAGCTCTCTACCGCCGTGGGCGTGCAGGGCCTGG TACTGGGGCAGTTTCGCGATCTTAACGATGCCGCCCTC GACCGTACCCCTGACGCTATCCTCAGCACCAACCACCT CAAGACCGGCATTCTGTTCAGCGCGATGCTGCAGATC GTCGCCATTGCTTCCGCCTCGTCGCCGAGCACGCGAGA GACGCTGCACGCCTTCGCCCTCGACTTCGGCCAGGCGT TTCAACTGCTGGACGATCTGCGTGACGATCACCCGGA AACCGGTAAAGATCGCAATAAGGACGCGGGAAAATCG ACGCTGGTCAACCGGCTGGGCGCAGACGCGGCCCGGC AAAAGCTGCGCGAGCATATTGATTCCGCCGACAAACA CCTCACTTTTGCCTGTCCGCAGGGCGGCGCCATCCGAC AGTTTATGCATCTGTGGTTTGGCCATCACCTTGCCGAC TGGTCACCGGTCATGAAAATCGCCTGA (SEQ ID NO: 2) | ¹ CGT to AGGA |
| | | ² read as TAA(stop) after mutation |
| Crtl | ATGAAAAAAACCGTTGTGATTGGCGCAGGCTTTGGTG GCCTGGCGCTGGCGATTCGCCTGCAGGCGGCAGGGAT CCCAACCGTACTGCTGGAGCAGCGGGACAAGCCCGGC GGTCGGGCCTACGTCTGGCATGACCAGGGCTTTACCTT TGACGCCGGGCCGACGGTGATCACCGATCCTACCGCG CTTGAGGCGCTGTTCACCCTGGCCGGCAGGCGCATGG AGGATTACGTCAGGCTGCTGCCGGTAAAACCCTTCTAC CGA¹CTCTGCTGGGAGTCCGGGAAGACCCTCGACTATG CTAA²CGACAGCGCCGAGCTTGAGGCGCAGATTACCCA GTTCAACCCCCGCGACGTCGAGGGCTACCGGCGCTTTC TGGCTTACTCCCAGGCGGTATTCCAGGAGGGATATTTG CGCCTCGGCAGCGTGCCGTTCCTCTCTTTTCGCGACAT GCTGCGCGCCGGGCCGCAGCTGCTTAAGCTCCAGGCG TGGCAGAGCGTCTACCAGTCGGTTTCGCGCTTTATTGA GGATGAGCATCTGCGGCAGGCCTTCTCGTTCCACTCCC TGCTGGTAGGCGGCAACCCCTTCACCACCTCGTCCATC TACACCCTGATCCACGCCCTTGAGCGGGAGTGGGGGG TCTGGTTCCCTGAGGGCGGCACCGGGGCGCTGGTGAA CGGCATGGTGAAGCTGTTTACCGATCTGGGCGGGGAG ATCGAACTCAACGCCCGGGTCGAAGAGCTGGTGGTGG CCGATAACCGCGTAAGCCAGGTCCGGCTGGCGGATGG TCGGATCTTTGACACCGACGCCGTAGCCTCGAACGCTG ACGTGGTGAACACCTATAAAAAGCTGCTCGGCCACCA TCCGGTGGGGCAGAAGCGGGCGGCAGCGCTGGAGCGC AAGAGCATGAGCAACTCGCTGTTTGTGCTCTACTTCGG CCTGAACCAGCCTCATTCCCAGCTGGCGCACCATACCA TCTGTTTTGGTCCCCGCTACCGGGAGCTGATCGACGAG ATCTTTACCGGCAGCGCGCTGGCGGATGACTTCTCGCT CTACCTGCACTCGCCCTGCGTGACCGATCCCTCGCTCG CGCCTCCCGGCTGCGCCAGCTTCTACGTGCTGGCCCCG GTGCCGCATCTTGGCAACGCGCCGCTGGACTGGGCGC AGGAGGGGCCGAAGCTGCGCGACCGCATCTTTGACTA CCTTGAAGAGCGCTATATGCCCGGCCTGCGTAGCCAG CTGGTGACCCAGCGGATCTTTACCCCGGCAGACTTCCA CGACACGCTGGATGCGCATCTGGGATCGGCCTTCTCCA TCGAGCCGCTGCTGACCCAAAGCGCCTGGTTCCGCCCG CACAACCGCGACAGCGACATTGCCAACCTCTACCTGG TGGGCGCAGGTACTCACCCTGGGGCGGGCATTCCTGG CGTAGTGGCCTCGGCGAAAGCCACCGCCAGCCTGATG ATTGAGGATCTGCAATGA (SEQ ID NO: 3) | ¹ CGA to AGGA |
| | | ² read as TAA(stop) after mutation |
| CrtB | ATGAGCCAACCGCCGCTGCTTGACCACGCCACGCAGA CCATGGCCAACGGCTCGAAAAGTTTTGCCACCGCTGC GAAGCTGTTCGACCCGGCCACCCGCCGTAGCGTGCTG ATGCTCTACACCTGGTGCCGCCACTGCGATGACGTCAT TGACGACCAGACCCACGGCTTCGCCAGCGAGGCCGCG GCGGAGGAGGAGGCCACCCAGCGCCTGGCCCGGCTGC GCACGCTGACCCTGGCGGCGTTTGAAGGGGCCGAGAT GCAGGATCCGGCCTTCGCTGCCTTTCAGGAGGTGGCGC TGACCCACGGTATTACGCCCCGCATGGCGCTCGATCAC CTCGACGGCTTTGCGATGGACGTGGCTCAGACCCGC¹T ATGTCACCTTTGA²GGATACGCTGCGCTACTGCTATCA CGTGGCGGGCGTGGTGGGTCTGATGATGGCCAGGGTG ATGGGCGTGCGGGATGAGCGGGTGCTGGATCGCGCCT GCGATCTGGGGCTGGCCTTCCAGCTGACGAATATCGCC CGGGATATTATTGACGATGCGGCTATTGACCGCTGCTA TCTGCCCGCCGAGTGGCTGCAGGATGCCGGGCTGACC CCGGAGAACTATGCCGCGCGGGAGAATCGGGCCGCGC TGGCGCGGGTGGCGGAGCGGCTTATTGATGCCGCAGA GCCGTACTACATCTCCTCCCAGGCCGGGCTACACGATC TGCCGCCGCGCTGCGCCTGGGCGATCGCCACCGCCCG CAGCGTCTACCGGGAGATCGGTATTAAGGTAAAAGCG GCGGGAGGCAGCGCCTGGGATCGCCGCCAGCACACCA GCAAAGGTGAAAAAATTGCCATGCTGATGGCGGCACC GGGGCAGGTTATTCGGGCGAAGACGACGAGGGTGACG CCGCGTCCGGCCGGTCTTTGGCAGCGTCCCGTTTAG (SEQ ID NO: 4) | ¹ CGC to AGGA |
| | | ² read as TAA(stop) after mutation |

The resulting expression cassette including P_{TRC}, lycopene pathway gene cluster, and terminator was amplified by PCR and ligated into the pGEM-T-Easy, resulting in pH.QLYC. To create the plasmid pQtrfA_oriV, trfA gene containing AGGA at 156R site under P_{J23110} was ordered as gBlock fragment, and we placed the fragment upstream of rrnB terminator in the pQGFPu, replacing the QGFP and QtRNAu expression cassettes.

**[Table 3]**

| **Gene** | **Sequence** | **Mutation/premat ure termination point** |
|---|---|---|
| trfA | ATGAATCGGACGTTTGACCGGAAGGCATACAGGCAAG AACTGATCGACGCGGGGTTTTCCGCCGAGGATGCCGA AACCATCGCAAGCCGCACCGTCATGCGTGCGCCCCGC GAAACCTTCCAGTCCGTCGGCTCGATGGTCCAGCAAG CTACGGCCAAGATCGAGCGCGACAGCGTGCAACTGGC TCCCCCTGCCCTGCCCGCGCCATCGGCCGCCGTGGAGC GTTCGCGTCGTCTCGAACAGGAGGCGGCAGGTTTGGC GAAGTCGATGACCATCGACACGCGAGGAACTATGACG ACCAAGAAGCGAAAAACCGCCGGCGAGGACCTGGCA AAACAGGTCAGCGAGGCCAAGCAGGCCGCGTTGCTGA AACACACGAAGCAGCAGATCAAGGAAATGCAGCTTTC CTTGTTCGATATTGCGCCGTGGCCGGACACGATGCGAG CGATGCCAAACGACACGGCCCGC1TCTGCCCTGTTCAC CACGCGC2AA3CAAGAAAATCCCGCGCGAGGCGCTGCA AAACAAGGTCATTTTCCACGTCAACAAGGACGTGAAG ATCACCTACACCGGCGTCGAGCTGCGGGCCGACGATG ACGAACTGGTGTGGCAGCAGGTGTTGGAGTACGCGAA GCGCACCCCTATCGGCGAGCCGATCACCTTCACGTTCT ACGAGCTTTGCCAGGACCTGGGCTGGTCGATCAATGG CCGGTATTACACGAAGGCCGAGGAATGCCTGTCGCGC CTACAGGCGACGGCGATGGGCTTCACGTCCGACCGCG TTGGGCACCTGGAATCGGTGTCGCTGCTGCACCGCTTC CGCGTCCTGGACCGTGGCAAGAAAACGTCCCGTTGCC AGGTCCTGATCGACGAGGAAATCGTCGTGCTGTTTGCT GGCGACCACTACACGAAATTCATATGGGAGAAGTACC GCAAGCTGTCGCCGACGGCCCGACGGATGTTCGACTA TTTCAGCTCGCACCGGGAGCCGTACCCGCTCAAGCTGG AAACCTTCCGCCTCATGTGCGGATCGGATTCCACCCGC GTGAAGAAGTGGCGCGAGCAGGTCGGCGAAGCCTGCG AAGAGTTGCGAGGCAGCGGCCTGGTGGAACACGCCTG GGTCAATGATGACCTGGTGCATTGCAAACGCTAG (SEQ ID NO: 5) | ¹ CGC to AGGA |
| | | ² C to T |
| | | ³ read as TAA(stop) after mutation |

From pBAC-lacZ, which was a gift from Keith Joung (Addgene plasmid #13422; http://n2t.net/addgene: 13422; RRID:Addgene_13422), oriV was cloned by PCR using primers set, ForiV and RoriV, then we replaced the ClodF 13 origin of replication by the oriV, yielding pQtrfA oriV. The Q-codon was restored with the original triplet codon by the site-directed mutagenesis, yielding pWTtrfA oriV. The plasmids and primer sequences used above are summarized in the table below:

**[Table 4]**

| **Plasmid** | | **Construction** |
|---|---|---|
| pGFP | Wild type GFP with silent mutation at 45L (CTG→CTT) | Mutation was introduced by PCR using Fgfp45Lmut and Rgfp45Lmut as primers and pCDF_sfGFP1as a template. Q5 Site-Directed Mutagenesis Kit (NEB) was used. |
| pQGFP | GFP containing the silent mutation at 45L (CTG→CTT) and Q-codon (AGGA) at 31R site (QGFP) | Q-codon, AGGA, was inserted by PCR using Fgfp31Rmut and Rgfp31Rmut as primers and pGFP as a template. Q5 Site-Directed Mutagenesis Kit (NEB) was used. |
| pQtRNAw | tRNA^{Arg} _{CCU} derived from *E. coli* tRNA^{Arg} _{CCU} (argW) | argW gene containing TCCT instead of CCT at the anticodon site was prepared by oligonucleotide dimerization, and inserted into pRF0G-Tyr (Addgene plasmid #113644; |
| | | http: //n2t.net/addgene: 113644; |
| | | RRID:Addgene_113644), replacing tRNA gene from *Methanococcus jannaschii,* by Gibson assembly. The backgroud vector was liniearized by PCR using the primers, FtRNAvectorW and RtRNAvectorW. |
| pQtRNAu | tRNA^{Arg} _{UCCU} derived from *E. coli* tRNA^{Arg} _{UCU} (argU) | argU gene containing TCCT instead of TCT at the anticodon site was prepared by oligonucleotide dimerization, and inserted into pRF0G-Tyr, replacing tRNA gene from *Methanococcus, jannaschii,* by Gibson assembly. The backgroud vector was liniearized by PCR using the primers, FtRNAvectorU and RtRNAvectorU. |
| pQGFPw | QGFP and QtRNAw | The expression cassette for QtRNAw from pQtRNAw was amplified by PCR using FpLPP and RrrnBTT and digested by BamHI and XhoI. The plasmid pQGFP was also digested by BamHI and XhoI. The QtRNAw expression cassette was inserted downstream of QGFP in the pQGFP by ligation. |
| ^{p}QGFPu | QGFP and QtRNAu | The expression cassette for QtRNAu from pQtRNAu was amplified by PCR using FpLPP and RrrnBTT and digested by BamHI and XhoI. The plasmid pQGFP was also digested by BamHI and XhoI. The QtRNAu expression cassette was inserted downstream of QGFP in the pQGFP by ligation. |
| pKmR | Wild type kanamycin resistance gene | Kanamycin resistance gene was inserted downstream of ClodF 13 origin in the pGFP, replacing the GFP expression cassette by Gibson assembly. Insert was amplified by PCR using primers, FkmR and RkmR, and the vector was linearized by PCR using primers, Fcdfvector and Rcdfvector. |
| pQKmR | Kanamycin resistance gene containing Q-codon (AGGA) at 12R | Q-codon, AGGA, was inserted at the 12R site of kanamycin resistance gene in the pKmR by PCR using FQkmR and RQkmR and followed by site-directed mutagenesis (NEB). |
| pLyc | Wild type CrtE, CrtI, and CrtB genes for lycopene production | Lycopene biosynthetic gene cluster from *Erwinia herbicola* was cloned by PCR from pAC-LYC (Addgene plasmid #53270; http://n2t.net/addgene:53270; RRID:Addgene_53270) using primers, FcrtE and RcrtB, and introduced to pGFP, replacing the GFP gene. |
| pQLyc | CrtE, CrtI and CrtB genes containing Q-codons (QcrtE, QcrtI and QcrtB) | Q-codon (AGGA) was introduced to three genes in the pLyc by site-directed mutagenesis (NEB). |
| pH.Qlyc | High copy plasmid for QcrtE, QcrtI and QcrtB expression | The expression cassette for QcrtE, QcrtI and QcrtB from pQLyc was amplified by PCR using Fptrc and RcrtB and ligated into pGEM-T-Easy vector. |
| pH. QtRNAw | High copy plasmid for QtRNAw expression | The expression cassette for QtRNAw from pQtRNAw was amplified by PCR using FpLPP and RrrnBTT and ligated into pGEM-T-Easy vector. |
| pH.QtRNAu | High copy plasmid for QtRNAu expression | The expression cassette for QtRNAu from pQtRNAu was amplified by PCR using FpLPP and RrrnBTT and ligated into pGEM-T-Easy vector. |
| pBAD-QtRNAu | QtRNAu expression under P_{BAD} | |
| pBAD-QtRNAw | QtRNAw expression under P_{BAD} | |
| pH.BAD-QtRNAw | High copy plasmid for QtRNAw expression under P_{BAD} | |

**[Table 5]**

| **Primer** | **Sequence** |
|---|---|
| Fgfp45Lmut | GACCCT**T**AAGTTCATCTGCACCACCGGCAAG (SEQ ID NO: 8) |
| Rgfp45Lmut | AGGGTCAGCTTGCCGTTGGTG (SEQ ID NO: 9) |
| Fgfp31Rmut | **AGGA**GGCGAGGGCGAGGGCGATG (SEQ ID NO: 10) |
| Rgfp31Rmut | CACGCTGAACTTGTGGCCGTTTACG (SEQ ID NO: 11) |
| QargW1 | GTCCTCTTAGTTAAATGGATATAACGAGCCCCT**TCCT**AAGGGC TAATTGCAGGTTCGATTCCTGCAGGGGACACCA*TTTATCACAGAT TG* (SEQ ID NO: 12) |
| QargW2 | *CAATCTGTGATAAA*TGGTGTCCCCTGCAGGAATCGAACCTGCAAT TAGCCCTT**AGGA**AGGGGCTCGTTATATCCATTTAACTAAGAGG AC (SEQ ID NO: 13) |
| QargU1 | GCGCCCTTAGCTCAGTTGGATAGAGCAACGACCT**TCCT**AAGTC GTGGGCCGCAGGTTCGAATCCTGCAGGGCGCGCCA*TTTATCACA GAT* (SEQ ID NO: 14) |
| QargU2 | *ATCTGTGATAAA*TGGCGCGCCCTGCAGGATTCGAACCTGCGGCC CACGACTT**AGGA**AGGTCGTTGCTCTATCCAACTGAGCTAAGGG CGC (SEQ ID NO: 15) |
| FtRNAvectorW | *GCGCGCCATTTATCACAGAT*TGGAAATTTTTGATCCTTAG (SEQ ID NO: 16) |
| RtRNAvectorW | *ACTGAGCTAAGGGCGCAATGCGTCTGATGGCAGC* (SEQ ID NO: 17) |
| FtRNAvectorU | *ACACCATTTATCACAGAT*TGGAAATTTTTGATCCTTAG (SEQ ID NO: 18) |
| RtRNAvectorU | *CATTTAACTAAGAGGACAATGCGTCTGATGGCAGC* (SEQ ID NO: 19) |
| FpLPP | CAAATGTAGCACCTGAAGTCAGC (SEQ ID NO: 20) |
| RrmBTT | CCGCTCGAGGGCATCGGTCGACTAAAAAAAATC (SEQ ID NO: 21) |
| FkmR | *TCTACTGAACCGCGGATCCCATATGGTCGACCTGCAGGC* (SEQ ID NO: 22) |
| RkmR | *CAGCCGGATCGGATCTGTAATCCGGGCAGC* (SEQ ID NO: 23) |
| Fcdfvector | *TTACAGATCCGATCCGGCTGCTAACAAAG* (SEQ ID NO: 24) |
| Rcdfvector | *CATATGGGATCCGCGGTTCAGTAGAAAAGATC* (SEQ ID NO: 25) |
| FQkmR | ACCGCGATTAAATTCCAACATGGATGCTG (SEQ ID NO: 26) |
| RQkmR | **TCCT**CGAGCAAGACGTTTCCCGTTGAATATG (SEQ ID NO: 27) |
| FcrtE | GAGATATACATATGGTGAGTGGCAGTAAAGC (SEQ ID NO: 28) |
| RctrB | CCGCTCGAGCTAAACGGGACGCTGCCAAAG (SEQ ID NO: 29) |
| Fptrc | TCTAGATTTCAGTGCTCCTTGAC (SEQ ID NO: 30) |

(In the table above, bold letters indicate mutations, italics indicate homologous regions, and underlines indicate restriction enzyme sites.)

### Example 3. Strain Construction

### 3-1. argW knockout

We knocked out argW from the genome of *E. coli* MG1655 using lambda (λ) Red recombineering. We first transformed pKD46 encoding λ Red recombinase into the strain and maintained its culture at 30°C. An overnight culture of MG1655 carrying pKD46 was diluted by 100-fold, and grown in 2-mL LB supplemented with carbenicillin until the cells reached an OD₆₀₀ of 0.5-0.6. The expression of the recombinase was induced by 0.2% L-arabinose after inoculation. The collected cells were repeatedly washed and concentrated with ice-cold, nuclease-free water to make them electrocompetent. A kanamycin resistance gene cassette flanked by flippase recognition target (FRT) sites was amplified from pKD4 by PCR using primers set containing 20-bp overhangs, which overlap with sequences located upstream and downstream of *argW* on the genome. The PCR product (~200 ng) was transformed, and after 2h incubation in 1-mL LB at 37°C, cells were spread onto LB agar plate supplemented with kanamycin to select kanamycin-resistant transformants. Integration of the kanamycin resistance gene into the targeted site on the genome was confirmed by colony PCR and sequence analysis. Next, pCP20 carrying a temperature-inducible flippase (FLP) gene was transformed, and cells were spread onto LB agar plate supplemented with chloramphenicol. After overnight culture at 30°C, a single colony was picked up and grown in 3-mL LB at 30°C until cells reached an OD₆₀₀ of 0.3, from which cells were grown at 37°C to induce FLP expression. 4h-incubation at 37 °C followed by dilution by 100-fold was repeated two times, and cells were spread onto the LB agar plate. After overnight culture at 37 °C, colonies were tested for loss of all antibiotic resistance by replica plating on LB plates containing carbenicillin, kanamycin, and chloramphenicol, respectively. Elimination of the kanamycin resistance gene was confirmed by colony PCR and sequence analysis.

### 3-2. Cos- and CRISPR Optimized MAGE Recombineerins: Cos-CRMAGE

To mutate AGGA to AGAA on 14 essential genes (lptF, dnaC, ftsL, lpxC, ispU, cdsA, rseP, tilS, mrdB, asnS, yceQ, asd, glyQ, folC), genome engineering was conducted by combining Cos-MAGE and CRMAGE methods, namely Cos-CRMAGE. As a starter cell, MG1655 *ΔargW* strain was transformed with pMA7CR_tetR, and the overnight culture was inoculated 1:1000 into 4-mL LB medium supplemented with carbenicillin (100 ug/mL) and grown at 37°C with shaking at 200 r.p.m. When cells reached an OD₆₀₀ of 0.5-0.6, L-arabinose at a final concentration of 0.2% was added to induce λ Red β protein expression. After 15 min induction, cells were moved to ice for 15 min, followed by centrifugation at 6,500 g for 5 min at 4°C. The supernatant was removed and the cell pellet was resuspended with 10 mL of ice-cold, nuclease-free water. Centrifugation and resuspension with 1 mL of ice-cold, nuclease-free water were repeated twice, and the final cell pellet was resuspended by 50 uL of ice-cold, nuclease-free water containing the appropriate MAGE oligonucleotide-pool (total concentration: -0.5-10 uM) and one of the self-killing plasmids (~250 ng), followed by electroporation (Biorad). Immediately, 950 uL of LB medium with carbenicillin was added into the electroporation cuvette and the cells were transferred to a new 15-mL culture tube (BD). After one hour recovery in LB medium supplemented with carbenicillin at 37°C, kanamycin was added and cells were grown for another two hours. Then, aTetracycline (aTc) was added to induce expression of Cas9 and sgRNA, and another two hours of cultivation was followed for negative selection. Cells were collected by centrifugation at 6,500 g for 5 min, and washed with fresh LB medium twice before transferred to 4-mL LB medium supplemented with carbenicillin, aTc (200 ng/mL), and L-rhamnose (0.2%) which is for plasmid curing, and overnight culture at 37°C. The entire process is defined as one cycle; an overnight culture of the previous cycle becomes a starter culture for the subsequent cycle. For each cycle, a plasmid encoding different sgRNA was employed, and two to four cycles, which consist of one round in Cos-CRMAGE, were conducted for each zone. At the final cycle, cells were grown for two hours in the LB medium containing carbenicillin, aTc, and L-rhamnose, and diluted and plated on the LB plate containing carbenicillin, aTc, and L-rhamnose.

Multiplex allele-specific colony PCR (MASC-PCR) was used to check mutation progress on target genes after each round. Briefly, two sets of primers were prepared for each mutation target. One forward primer ends with AGG, and the other forward primer ends with AGA, corresponding to wild type (WT) allele or mutant (MUT) allele, respectively. For both WT and MUT alleles, the same reverse primer was used. For mrdB, yceQ, and folC, which the target AGG is located nearly at the end of the gene, the discrimination was made for the reverse primers, and the same forward primers were used for both WT and Mut alleles. At an optimal Tₘ, depending on the mutation progress, either WT primer or MUT primer results in the wobble base pairing at the end, inhibiting extension by PCR and resulting in no band in a gel. When designing primer pairs for MASC-PCR, we aimed for their annealing temperature to be 62°C or 64°C, letting their length to be at least 17 nucleotides. Using a gradient PCR, for each mutation target, we optimized Tₘ, which resulted in an expected band with primers for WT allele but not with primers for MUT allele, with the WT clone. We also intended for each PCR to be resulted in bands of different lengths. Accordingly, we grouped genes by its optimal Tₘ and expected band length, and PCRs for genes in the same group were conducted in a single PCR tube using *Accupower^{®}* Multiplex PCR PreMix from Bioneer. After running on 1% agarose gel, PCR products showed expected bands with MUT primer sets were followed by sequence analysis. The colony containing maximal mutations was chosen as a starter cell for the next round of Cos-CRMAGE. In the next round, we added MAGE oligonucleotides for the next zone and also those for the previous zone when it was incomplete. The cycles were repeated until we found a final mutant strain containing AGA instead of AGG at all targeted sites, namely MUT.

The plasmids and primer sequences used in the above experiments are summarized in the table below:

**[Table 6]**

| **Plasmid** | | **Construction** |
|---|---|---|
| pMA7CR_2.0_ tetR | CRISPR-Cas9 and λ/RED b-protein | TetR gene was cloned by PCR using FtetR and RtetR, and inserted into pMA7CR_2.0 (Addgene plasmid #73950; http://n2t.net/addgene:73950; RRID:Addgene_73950) by Gibson assembly. |
| pMAZ-SK1 | lptF-mutant targeting sgRNA and self-eliminating circuit | DNA fragment encoding lptF-mutant targeting sgRNA was prepared by oligonucleotide dimerization, and inserted into pMAZ-SK by Gibson assembly. |
| pMAZ-SK2 | dnaC-mutant targeting sgRNA and self-eliminating circuit | DNA fragment encoding dnaC-mutant targeting sgRNA was prepared by oligonucleotide dimerization, and inserted into pMAZ-SK by Gibson assembly. |
| pMAZ-SK3 | ftsL-mutant targeting sgRNA and self-eliminating circuit | DNA fragment encoding ftsL-mutant targeting sgRNA was prepared by oligonucleotide dimerization, and inserted into pMAZ-SK by Gibson assembly. |
| pMAZ-SK4 | lpxC-mutant targeting sgRNA and self-eliminating circuit | DNA fragment encoding lpxC-mutant targeting sgRNA was prepared by oligonucleotide dimerization, and inserted into pMAZ-SK by Gibson assembly. |
| pMAZ-SK5 | ispU-mutant targeting sgRNA and self-eliminating circuit | DNA fragment encoding ispU-mutant targeting sgRNA was prepared by oligonucleotide dimerization, and inserted into pMAZ-SK by Gibson assembly. |
| pMAZ-SK6 | cdsA-mutant targeting sgRNA and self-eliminating circuit | DNA fragment encoding cdsA-mutant targeting sgRNA was prepared by oligonucleotide dimerization, and inserted into pMAZ-SK by Gibson assembly. |
| pMAZ-SK7 | rseP-mutant targeting sgRNA and self-eliminating circuit | DNA fragment encoding rseP-mutant targeting sgRNA was prepared by oligonucleotide dimerization, and inserted into pMAZ-SK by Gibson assembly. |
| pMAZ-SK8 | tilS-mutant targeting sgRNA and self-eliminating circuit | DNA fragment encoding tilS-mutant targeting sgRNA was prepared by oligonucleotide dimerization, and inserted into pMAZ-SK by Gibson assembly. |
| pMAZ-SK9 | mrdB-mutant targeting sgRNA and self-eliminating circuit | DNA fragment encoding mrdB-mutant targeting sgRNA was prepared by oligonucleotide dimerization, and inserted into pMAZ-SK by Gibson assembly. |
| pMAZ-SK10 | asnS-mutant targeting sgRNA and self-eliminating circuit | DNA fragment encoding asnS-mutant targeting sgRNA was prepared by oligonucleotide dimerization, and inserted into pMAZ-SK by Gibson assembly. |
| pMAZ-SK11 | yceQ-mutant targeting sgRNA and self-eliminating circuit | DNA fragment encoding yceQ-mutant targeting sgRNA was prepared by oligonucleotide dimerization, and inserted into pMAZ-SK by Gibson assembly. |
| pMAZ-SK12 | asD-mutant targeting sgRNA and self-eliminating circuit | DNA fragment encoding asD-mutant targeting sgRNA was prepared by oligonucleotide dimerization, and inserted into pMAZ-SK by Gibson assembly. |
| pMAZ-SK13 | glyQ-mutant targeting sgRNA and self-eliminating circuit | DNA fragment encoding glyQ-mutant targeting sgRNA was prepared by oligonucleotide dimerization, and inserted into pMAZ-SK by Gibson assembly. |
| pMAZ-SK14 | folC-mutant targeting sgRNA and self-eliminating circuit | DNA fragment encoding folC-mutant targeting sgRNA was prepared by oligonucleotide dimerization, and inserted into pMAZ-SK by Gibson assembly. |

**[Table 7]**

| **Oligonucleotide** | **Sequence** |
|---|---|
| MAGE lptF | A*C*CAGATTCGCCGGAATATCGCCGTCAACCGCTGCGC CGAGGATTCTCACTAACTTTTGACAGAAGAAGATCAAA AGCAAGATGAAGAGT (SEQ ID NO: 31) |
| MAGE dnaC | T*C*ATCACCGTGGCCGATATTATGTCGGCGATGAAAGA |
| | TACCTTCAGAAATAGCGGTACCAGCGAAGAACAACTGC TTAACGATCTGAGCA (SEQ ID NO: 32) |
| MAGE_ftsL | T*T*TTGCACTACGATATTTTCTTGTGACGGATCAACATG CTGCATTTGCAGCTTTTCCGTGGCGATTCTTTCCACCCG GCTATGGTCGCCG (SEQ ID NO: 33) |
| MAGE lpxC | G*T*ATGTAAACCGACACCCGTCGCCTGAACGATACGTT TAAGTGTTCTTTGTTTGATCATCGTATTATCTCGCCAAA TTACCTATCCAACC (SEQ ID NO: 34) |
| MAGE i spU | A*T*TTGCCAAAGCAAAAAGTTACTAATGCGATGCTCCC CCCCAGTTCTAATTACTAAATCTACAGGGGCCAGTTCA TGCATACAGACATGC (SEQ ID NO: 35) |
| MAGE_cdsA | C*A*AATCCCAGAGAAAACTCAGCATATTACCTTCCGTT AAAGCGTTCTGAATACCAGTAACAACAAGCAAGCAAA GACCGGTACCGCAGCC (SEQ ID NO: 36) |
| MAGE_rseP | A*A*GGTCACCCACTGCGTTAAGGGCTGACCATCGACTT TAACGATTCTGTCGCCTGCTTGCAAACCTGCCTTGCTTG CCGCCGAGTTTGGC (SEQ ID NO: 37) |
| MAGE_tilS | C*G*TAAACAGGGTGAGGCATCTTCCCGCGCCAGCGCC ACTTCCTGCCAGATTCTCACCAACGCGTCGCGGGAAGG CATCGGTGCATTCTGC (SEQ ID NO: 38) |
| MAGE_mrdB | T*G*CTGATGGCTGGGTTCGGGATTGTAATGTCAATCCA CACCCACAGAAAAATGTTGTCGAAAAGCGTGTAAGAG GTGCGCAATGCGTAAG (SEQ ID NO: 39) |
| MAGE_asnS | A*G*GTGGATTATACCGACGCAGTGACCATTCTCGAAAA CTGCGGCAGAAAGTTTGAAAACCCGGTTTACTGGGGAG TCGATCTCTCTTCTG (SEQ ID NO: 40) |
| MAGE_yceQ | T*A*ACAAGGCTTGCAGTGGAATAATGAGGCCGTTTCCG TGTCCATTCTTGTTAAAACAAGAAATTTTACGGAATAA CCCATTTTGCCCGAC (SEQ ID NO: 41) |
| MAGE_asd | G*A*CATCAACATCAGGCTTACGGTACAGTTACCGCCAA CAAAAGTTCTGATGCCATTATTTAATCCGTCGGTAATG ACGTCCTGATTGACG (SEQ ID NO: 42) |
| MAGE_glyQ | C*G*AGCCCAGTAATCCTGTAAGGTCAGGATCAAGCCCT GGAAGGTTCTGGTATCAAACTTTTGCATATTATTTCGTG CTGGATACGTGTGG (SEQ ID NO: 43) |
| MAGE folC | T*C*GTGCCCACTAACCGATTCTGAAACTTACTTGCCAC CGCTTCTTCTCGCGTCAATCACTTCCATGACATGTGCGA CCGTGTGGAAAGAA (SEQ ID NO: 44) |
| FlptFW | CTTTTGATCTTCTTCTGTCAAAAGTTAGTGAGG (SEQ ID NO: 45) |
| FlptFMT | CTTTTGATCTTCTTCTGTCAAAAGTTAGTGAGA (SEQ ID NO: 46) |
| RlptF | ACAGGCTTAATGGCAGGATAAGC (SEQ ID NO: 47) |
| FdnaCW | GGCGATGAAAGATACCTTCAGG (SEQ ID NO: 48) |
| FdnaCMT | GGCGATGAAAGATACCTTCAGA (SEQ ID NO: 49) |
| RdnaC | TCTTTACCTGTTACCCGGCTAC (SEQ ID NO: 50) |
| FftsLW | CATAGCCGGGTGGAAAGG (SEQ ID NO: 51) |
| FftsLMT | CATAGCCGGGTGGAAAGA (SEQ ID NO: 52) |
| RtfsL | CAGCGCCAGGAGAATACAG (SEQ ID NO: 53) |
| FlpxCW | GCGAGATAATACGATGATCAAACAAAGG (SEQ ID NO: 54) |
| FlpxCMT | GCGAGATAATACGATGATCAAACAAAGA (SEQ ID NO: 55) |
| RlpxC | CATGACCGGATTTATAAGCGGTAAATG (SEQ ID NO: 56) |
| FispUW | TGGCCCCTGTAGATTTAGTAATTAGG (SEQ ID NO: 57) |
| FispUMT | TGGCCCCTGTAGATTTAGTAATTAGA (SEQ ID NO: 58) |
| RispU | AGAAGGGAACAATGGTTAGCAC (SEQ ID NO: 59) |
| FcdsAW | CTTGCTTGTTGTTACTGGTATTCAGG (SEQ ID NO: 60) |
| FcdsAMT | CTTGCTTGTTGTTACTGGTATTCAGA (SEQ ID NO: 61) |
| RcdsA | CGAGTTTGGCTGCACATTTTC (SEQ ID NO: 62) |
| FrsePW | TTGCAAGCAGGCGACAGG (SEQ ID NO: 63) |
| FrsePMT | TTGCAAGCAGGCGACAGA (SEQ ID NO: 64) |
| RrseP | TCGATCGCAAGGAACAGCAG (SEQ ID NO: 65) |
| FtilSW | GCGACGCGTTGGTGAGG (SEQ ID NO: 66) |
| FtilSMT | GCGACGCGTTGGTGAGA (SEQ ID NO: 67) |
| RtilS | GCAATCAGCGTTTCGCCAT (SEQ ID NO: 68) |
| FmrdB | GCTGGACCTCGGTATTGTTC (SEQ ID NO: 69) |
| RmrdBW | GTTACACGCTTTTCGACAACATTTTC (SEQ ID NO: 70) |
| RmrdBMT | GTTACACGCTTTTCGACAACATTTTT (SEQ ID NO: 71) |
| FasnSW | CTCGAAAACTGCGGCAGG (SEQ ID NO: 72) |
| FasnSMT | CTCGAAAACTGCGGCAGA (SEQ ID NO: 73) |
| RasnS | ACCAAGACCGAAACCTGAATGC (SEQ ID NO: 74) |
| FyceQ | TGTCGGTTGCCCGTTTTTC (SEQ ID NO: 75) |
| RyceQW | GCCGTTTCCGTGTCCATC (SEQ ID NO: 76) |
| RyceQMT | GCCGTTTCCGTGTCCATT (SEQ ID NO: 77) |
| FasDW | CGACGGATTAAATAATGGCATCAGG (SEQ ID NO: 78) |
| FasDMT | CGACGGATTAAATAATGGCATCAGA (SEQ ID NO: 79) |
| RasD | CGATCGTTCGGAACGACTTTC (SEQ ID NO: 80) |
| FglyQW | CACGAAATAATATGCAAAAGTTTGATACCAGG (SEQ ID NO: 81) |
| FglyQMT | CACGAAATAATATGCAAAAGTTTGATACCAGA (SEQ ID NO: 82) |
| RglyQ | CGAAGTTGTAAGTGGACTGCTC (SEQ ID NO: 83) |
| FfolC | TGGAAGTCAGTGAAAATGCCATTC (SEQ ID NO: 84) |
| RfolCW | CTTACTTGCCACCGCTTCTC (SEQ ID NO: 85) |
| RfolCMT | CTTACTTGCCACCGCTTCTT (SEQ ID NO: 86) |
| FsgRNAvec | GTTTTAGAGCTAGAAATAGCAAGTTAAAATAAG (SEQ ID NO: 87) |
| RsgRNAvec | GTGCTCAGTATCTCTATCACTGATAG (SEQ ID NO: 88) |
| sg_lptF1 | CAGTGATAGAGATACTGAGCACTCTTCTGTCAAAAGTTAG TGGTTTTAGAGCTAGAAATAGCAAG (SEQ ID NO: 89) |
| sg_lptF2 | CTTGCTATTTCTAGCTCTAAAACCACTAACTTTTGACAGAA GAGTGCTCAGTATCTCTATCACTG (SEQ ID NO: 90) |
| sg_dnaC1 | CAGTGATAGAGATACTGAGCACCGGCGATGAAAGATACC TTCGTTTTAGAGCTAGAAATAGCAAG (SEQ ID NO: 91) |
| sg_dnaC2 | CTTGCTATTTCTAGCTCTAAAACGAAGGTATCTTTCATCGC CGGTGCTCAGTATCTCTATCACTG (SEQ ID NO: 92) |
| sg_ftsL1 | CAGTGATAGAGATACTGAGCACGCGACCATAGCCGGGTG GAAGTTTTAGAGCTAGAAATAGCAAG (SEQ ID NO: 93) |
| sg_ftsL2 | CTTGCTATTTCTAGCTCTAAAACTTCCACCCGGCTATGGTC GCGTGCTCAGTATCTCTATCACTG (SEQ ID NO: 94) |
| sg_lpxC1 | CAGTGATAGAGATACTGAGCACATAATACGATGATCAAA CAAGTTTTAGAGCTAGAAATAGCAAG (SEQ ID NO: 95) |
| sg_lpxC2 | CTTGCTATTTCTAGCTCTAAAACTTGTTTGATCATCGTATT ATGTGCTCAGTATCTCTATCACTG (SEQ ID NO: 96) |
| sg_ispU1 | CAGTGATAGAGATACTGAGCACCCCCTGTAGATTTAGTAA TTGTTTTAGAGCTAGAAATAGCAAG (SEQ ID NO: 97) |
| sg_ispU2 | CTTGCTATTTCTAGCTCTAAAACAATTACTAAATCTACAGG GGGTGCTCAGTATCTCTATCACTG (SEQ ID NO: 98) |
| sg_cdsA1 | CAGTGATAGAGATACTGAGCACGCTTGTTGTTACTGGTAT TCGTTTTAGAGCTAGAAATAGCAAG (SEQ ID NO: 99) |
| sg_cdsA2 | CTTGCTATTTCTAGCTCTAAAACGAATACCAGTAACAACA AGCGTGCTCAGTATCTCTATCACTG (SEQ ID NO: 100) |
| sg_rseP1 | CAGTGATAGAGATACTGAGCACCAGGTTTGCAAGCAGGC GACGTTTTAGAGCTAGAAATAGCAAG (SEQ ID NO: 101) |
| sg_rseP2 | CTTGCTATTTCTAGCTCTAAAACGTCGCCTGCTTGCAAACC TGGTGCTCAGTATCTCTATCACTG (SEQ ID NO: 102) |
| sg_tilS1 | CAGTGATAGAGATACTGAGCACCTTCCCGCGACGCGTTGG TGGTTTTAGAGCTAGAAATAGCAAG (SEQ ID NO: 103) |
| sg_tilS2 | CTTGCTATTTCTAGCTCTAAAACCACCAACGCGTCGCGGG AAGGTGCTCAGTATCTCTATCACTG (SEQ ID NO: 104) |
| sg_mrdB1 | CAGTGATAGAGATACTGAGCACTAATGTCAATCCACACCC ACGTTTTAGAGCTAGAAATAGCAAG (SEQ ID NO: 105) |
| sg_mrdB2 | CTTGCTATTTCTAGCTCTAAAACGTGGGTGTGGATTGACAT TAGTGCTCAGTATCTCTATCACTG (SEQ ID NO: 106) |
| sg_asnS1 | CAGTGATAGAGATACTGAGCACCCATTCTCGAAAACTGCG GCGTTTTAGAGCTAGAAATAGCAAG (SEQ ID NO: 107) |
| sg_asnS2 | CTTGCTATTTCTAGCTCTAAAACGCCGCAGTTTTCGAGAAT GGGTGCTCAGTATCTCTATCACTG (SEQ ID NO: 108) |
| sg_yceQ1 | CAGTGATAGAGATACTGAGCACTAAAATTTCTTGTTTTAA CAGTTTTAGAGCTAGAAATAGCAAG (SEQ ID NO: 109) |
| sg_yceQ2 | CTTGCTATTTCTAGCTCTAAAACTGTTAAAACAAGAAATTT TAGTGCTCAGTATCTCTATCACTG (SEQ ID NO: 110) |
| sg_asD1 | CAGTGATAGAGATACTGAGCACACGGATTAAATAATGGC ATCGTTTTAGAGCTAGAAATAGCAAG (SEQ ID NO: 111) |
| sg_asD2 | CTTGCTATTTCTAGCTCTAAAACGATGCCATTATTTAATCC GTGTGCTCAGTATCTCTATCACTG (SEQ ID NO: 112) |
| sg_glyQ1 | CAGTGATAGAGATACTGAGCACATATGCAAAAGTTTGATA CCGTTTTAGAGCTAGAAATAGCAAG (SEQ ID NO: 113) |
| sg_glyQ2 | CTTGCTATTTCTAGCTCTAAAACGGTATCAAACTTTTGCAT ATGTGCTCAGTATCTCTATCACTG (SEQ ID NO: 114) |
| sg_folC1 | CAGTGATAGAGATACTGAGCACTCATGGAAGTGATTGAC GCGGTTTTAGAGCTAGAAATAGCAAG (SEQ ID NO: 115) |
| sg_folC2 | CTTGCTATTTCTAGCTCTAAAACCGCGTCAATCACTTCCAT GAGTGCTCAGTATCTCTATCACTG (SEQ ID NO: 116) |

(In the table above, * indicates the phosphorothioate linkages at the 5' end, bold letters indicate mutations, italics indicate homologous regions, and underlines indicate enzyme sites.)

**3-3.** 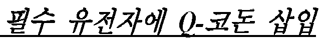

To prevent leakage of the engineered strain, a process of inserting a Q-codon into an essential gene was performed. The CRMAGE method used in Example 3-2 above was used, and two types of target essential genes, cysS and fldA, were selected *(cysS:* 55R*, f7dA:* 106R).

**[Table 8]**

| **Gene** | **Sequence** | **Mutation/premat ure termination point** |
|---|---|---|
| cysS | ATGCTAAAAATCTTCAATACTCTGACACGCCAAAAAG AGGAATTTAAGCCTATTCACGCCGGGGAAGTCGGCAT GTACGTGTGTGGAATCACCGTTTACGATCTCTGTCATA TCGGTCACGGGCGTACCTTTGTTGCTTTTGACGTGGTT GCGCGCTATCTGCGT¹TTCCTCGGCTATAA²ACTGAAGT ATGTGCGCAACATTACCGATATCGACGACAAAATCAT CAAACGCGCCAATGAAAATGGCGAAAGCTTTGTGGCG ATGGTGGATCGCATGATCGCCGAAATGCACAAAGATT TTGATGCTTTGAACATTCTGCGCCCGGATATGGAGCCG CGCGCGACGCACCATATCGCAGAAATTATTGAACTCA CTGAACAACTGATCGCCAAAGGTCACGCTTATGTGGC GGACAACGGCGACGTGATGTTCGACGTCCCGACCGAT CCAACTTATGGCGTGCTGTCGCGTCAGGATCTCGACCA GCTGCAGGCAGGCGCGCGCGTTGACGTGGTCGACGAC AAACGCAACCCAATGGACTTCGTTCTGTGGAAGATGT CGAAAGAGGGCGAACCGAGCTGGCCGTCTCCGTGGGG CGCGGGTCGTCCTGGCTGGCACATTGAATGTTCGGCAA TGAACTGCAAGCAGCTGGGTAACCACTTTGATATCCAC GGCGGCGGTTCAGACCTGATGTTCCCGCACCACGAAA ACGAAATCGCGCAGTCCACCTGTGCCCATGATGGTCA GTATGTGAACTACTGGATGCACTCGGGGATGGTGATG GTTGACCGCGAGAAGATGTCCAAATCGCTGGGTAACT TCTTTACCGTGCGCGATGTGCTGAAATACTACGACGCG GAAACCGTGCGTTACTTCCTGATGTCGGGCCACTATCG CAGCCAGTTGAACTACAGCGAAGAGAACCTGAAGCAG GCGCGTGCGGCGCTGGAGCGTCTCTACACTGCGCTGC GCGGCACAGATAAAACCGTTGCGCCTGCCGGTGGCGA AGCGTTTGAAGCGCGCTTTATTGAAGCGATGGACGAC GATTTCAACACCCCGGAAGCCTATTCCGTACTGTTTGA TATGGCGCGTGAAGTAAACCGTCTGAAAGCAGAAGAT ATGGCAGCGGCGAATGCAATGGCATCTCACCTGCGTA AACTTTCCGCTGTATTGGGCCTGCTGGAGCAAGAACCG GAAGCGTTCCTGCAAAGCGGCGCGCAGGCAGACGACA GCGAAGTGGCTGAGATTGAAGCGTTAATTCAACAGCG TCTGGATGCCCGTAAAGCGAAAGACTGGGCGGCGGCG GATGCGGCGCGTGATCGTCTTAACGAGATGGGGATCG TGCTGGAAGATGGCCCGCAAGGGACCACCTGGCGTCG TAAGTAA (SEQ ID NO: 6) | ¹ CGT to AGGA |
| | | ² read as TAA(stop) after mutation |
| fldA | ATGGCTATCACTGGCATCTTTTTCGGCAGCGACACCGG TAATACCGAAAATATCGCAAAAATGATTCAAAAACAG CTTGGTAAAGACGTTGCCGATGTCCATGACATTGCAAA AAGCAGCAAAGAAGATCTGGAAGCTTATGACATTCTG CTGCTGGGCATCCCAACCTGGTATTACGGCGAAGCGC AGTGTGACTGGGATGACTTCTTCCCGACTCTCGAAGAG ATTGATTTCAACGGCAAACTGGTTGCGCTGTTTGGTTG TGGTGACCAGGAAGATTACGCCGAATATTTCTGCGAC GCATTGGGCACCATCCGC¹GACATCATTGA²ACCGCGC GGTGCAACCATCGTTGGTCACTGGCCAACTGCGGGCT ATCATTTCGAAGCATCAAAAGGTCTGGCAGATGACGA CCACTTTGTCGGTCTGGCTATCGACGAAGACCGTCAGC CGGAACTGACCGCTGAACGTGTAGAAAAATGGGTTAA ACAGATTTCTGAAGAGTTGCATCTCGACGAAATTCTCA ATGCCTGA (SEQ ID NO: 7) | ¹ CGC to AGGA |
| | | ² read as TGA(stop) after mutation |

Gene *cysS* is an essential gene encoding cysteine-tRNA ligase, and *fldA* is an essential gene encoding flavodoxin 1. During the above CRMAGE process, Q-tRNA was placed under constitutive promoter, allowing the strain to survive even if the Q-codon is inserted into an essential gene. After the Q-codon was successfully replaced, the "constitutively expressed Q-tRNA was replaced with an inducible Q-tRNA.

### 3-4. Plasmid Transformation

The plasmids, pGFP, pQGFP, pQGFPw and pQGFPu, were transformed into *E.coli* MG1655, yielding four different strains which include a positive control with no Q-codon, negative control with Q-codon but no Q-tRNAs, and two strains containing both Q-codon and Q-tRNA (QtRNAw or QtRNAu, respectively).

### Example 4. Analyses

### 4-1. GFP fluorescence measurement

Strains for GFP or QGFP expression were grown overnight in 2-mL LB medium supplemented with kanamycin at 37 °C, shaking at 200 r.p.m. Cells were diluted to OD₆₀₀ of 0.02 with a fresh LB medium containing kanamycin, and 100 uL of culture was transferred onto a 96-well black plate (Corning). Cells were grown at 37°C, shaking at 600 r.p.m in a plate shaker (OHAUS). After two hours, protein expression was induced with 1 mM of IPTG. Cell density (OD₆₀₀) and GFP fluorescence were measured every hour using a multiplate reader (Hidex), and the fluorescence was normalized to OD₆₀₀.

### 4-2. SDS-PAGE

Strains for protein expression were grown overnight in 2-mL LB medium supplemented with appropriate antibiotics at 37°C, shaking at 200 r.p.m. Cells were diluted to OD₆₀₀ of 0.05 with a fresh 4-mL LB medium containing the antibiotics and grown 37°C, shaking at 200 r.p.m. After two hours, target protein and Q-tRNA expression were induced with 1 mM of IPTG and 0.2% arabinose, respectively, and followed by 4-6 hours incubation at 37°C shaking at 200 r.p.m. Cells were harvested by centrifugation at 4,000 r.p.m for 20 min at 4°C, and the cell pellets were stored at -80°C. On the next day, cell pellets were thawed at room temperature and resuspended in 400 ul of cell lysis buffer (B7435, Sigma-Aldrich). After 15 min, cell lysates were centrifuged at 13,500 r.p.m for 5 min. The supernatant containing soluble protein fraction was carefully transferred to a new tube. The pellets containing insoluble protein fraction were resuspended in 400 ul of PBS buffer containing 8 M urea. After 30 min incubation with gentle mixing, the insoluble fraction was collected by centrifugation at 13,500 r.p.m for 5 min. Each sample was mixed with 2X Tris-glycine SDS sample buffer (LC2676, Invitrogen) and heated at 95 °C for 5 min. 20 ul of each sample was loaded into Novex 16% Tricine mini protein gel (EC6695BOX), and the gel was run at 200V for 45-60 min. The run gel was stained with coomassie blue for 1-2 hours with gentle mixing, and destained for >2 hours.

### 4-3. MALDI-TOF

To identify the protein, trypsin digestion was followed by MALDI-TOF analysis (Microflex LRF 20, Bruker Daltonics). Spectra were obtained at 300 shots/spectrum, and peaks were generated through Flex Analysis 3.0. The thresholds for obtaining peaks are: 500 for minimal resolution of monoisotopic mass, 6 for S/N. The above MALDI-TOF process was conducted by Genomine Co., Ltd.

### 4-4. tREX analysis

Strains for tREX analysis were grown in 2-mL LB medium supplemented with appropriate antibiotics overnight at 37°C, shaking at 200 r.p.m. The overnight culture was diluted with 50 mL 2xYT medium containing the antibiotics and grown at 37°C. When the OD₆₀₀ reached 0.5-1, cells were collected by centrifugation for 10 min at 3,500 r.p.m. and room temperature, and the pellet was resuspended with 800-µL buffer D (150 mM NaCl, 50 mM sodium acetate (pH5), 10 mM MgCl₂ and 0.1 mM EDTA). After another centrifugation for 2 min at 7,400 r.p.m., the pellet was resuspended with 450-µL buffer D. For cell lysis, 50-µL liquified phenol was added to cells, followed by head-over-tail rotation for 15 min at 15 r.p.m. The supernatant from centrifugation at 14,500 r.p.m. for 25 min at 4°C was mixed with 500-µL chloroform by vigorous vortexing for 1 min. The following centrifuge at 14,500 r.p.m. for 1 min resulted in two-layer separation, and the top layer containing tRNAs (~480 µL) was transferred to a new tube. For tREX analysis, the tRNA extracts were processed in three different ways: 1) 136-µL tRNA extract was mixed with 40-µL buffer D and precipitated with ethanol by 1 h incubation at 4°C, followed by removal of supernatant after centrifugation at 14,500 r.p.m. for 25 min and drying. The precipitated sample was resuspended with buffer D to a final concentration of 1 µg/µL (Biodrop), resulting in positive control for full extension, 2) 136-µL tRNA extract was mixed with 8 µL of 600 mM NaOH and incubated for 1h at 42°C. Each 16 µL of 3 M sodium acetate (pH5.2) and 100 mM NaIO₄ were added and followed by 1.5 h further incubation. Ethanol precipitation was conducted as previously, resulting in negative control for no extension, and 3) 136-µL tRNA extract was mixed with 24 µL of buffer D and 16 µL of 100 mM NaIO₄. After 1.5h incubation at room temperature, the sample was precipitated with ethanol as previously, resulting in a testing sample. To confirm probe specificity, tRNA extract from untransformed MG1655 *ΔargW* strain was processed in the same way as a testing sample. For extension reaction, 2 µL of processed tRNAs were mixed with 5 µL of NEBuffer 2.1, 1 µL of dNTPs (10 mM each), 1 µL of Cy5-labeled DNA probe (1 µM, Bioneer), and 40.5 µL of nuclease-free water. The annealing reactions were conducted in a thermocycler (Applied biosystems) with the following settings: 95°C for 1 min, 70°C for 2 min, 50°C for 2 min, and followed by incubation at 4°C. Then, 0.5 µL of Klenow Fragment with no 3'->5' exonuclease activity (M0212S, NEB) was added and followed by 20 min incubation at 37°C. To confirm the extension, each reaction mixed with 2x loading dye (8M urea and 0.04% Orange G) was run on 8% PAGE gel (acrylamide (19:1)) at 200V for 45 min. After staining (SYBR Gold Nucleic Acid Gel Stain, Invitrogen), gel was imaged on Azure600^{®}(700nm).

### 4-5. Lycopene measurement

Strains for lycopene production were grown overnight in 2-mL LB medium supplemented with appropriate antibiotics at 37°C, shaking at 200 r.p.m. Cells were diluted to OD₆₀₀ of 0.05 with a fresh 4-mL LB medium containing the antibiotics and grown 37°C, shaking at 200 r.p.m. After two hours, lycopene production pathway and Q-tRNA expression were induced with 1 mM of IPTG and 0.2% arabinose, respectively, and followed by overnight incubation at 30°C shaking at 200 r.p.m. Cells were collected.

### 4-6. Essential gene test

Two plasmids, containing Q-trfA and QtRNAw expression cassettes, respectively, were co-transformed into DH5α (Enzynomics, CP010). After 1-h recovery in 1-mL LB medium, cells were spread onto two separate plates: 1) one contains kanamycin and arabinose (0.2%) and 2) the other contains only kanamycin. Cells on plates were imaged on Azure 600^{®} (True color imaging). Colonies from the plate containing kanamycin and arabinose were picked up and grown overnight in 2-mL LB medium supplemented with kanamycin and arabinose. The overnight culture was diluted in 1: 1,000 with a fresh 2-mL LB medium and further grown for 12 hours without arabinose induction. Then, cells were diluted in 1:20,000 with a fresh LB medium containing kanamycin with or without arabinose. 100 uL of culture was transferred onto a 96-well black plate (CLS3904, Corning), and cells were grown at 37°C, shaking at 600 r.p.m in a plate shaker (OHAUS). Cell growth was monitored by measuring cell density (OD₆₀₀) every two hour for 16 hours (Hidex).

Strains that were engineered to contain Q-codon on one of the essential genes were transformed with the plasmid containing QtRNAw expression cassette. The overnight culture of each strain was diluted in 1 :20,000 with a fresh LB medium containing carbenicillin and cultured in a 96-well black plate at 37°C and 600 r.p.m. As a strain survival signal, anhydrotetracycline (ATc) was used at 50ng/mL. Cell growth was monitored by measuring cell density (OD₆₀₀) every two hour for 16 hours (Hidex). Then, the overnight culture was serially diluted in PBS buffer over a 6-log range and 5µL of each dilution was spotted onto LB agar plates containing carbenicillin and ATc, and the plates were imaged on Azure 600^{®} (True color imaging).

### 4-7. Lactoferricin production

For lactoferricin production analysis, an overnight culture was inoculated into a fresh LB medium containing appropriate antibiotics at an initial OD₆₀₀ of 0.05. Gene expression was induced by 1 mM of IPTG and 0.2% arabinose when the OD₆₀₀ reached 0.4. This was followed by a 12-hour cultivation. To confirm the correct translation, SDS-PAGE (NovexTM 16% Tricine, Invitrogen, #EC6695BOX) was performed. For protein identification, protein spots were excised, digested with trypsin (Pierce^{™} Trypsin Protease, MS Grade, 90058; Thermofisher) and subjected to MALDI-TOF analysis (ABsciex TripleTOF 5600+, Sciex). Analyst^{®} TF 1.7.1 (operation), Proteinpilot^{tm} 4.5 (sequence analysis), and PeakView 2.20 (chromatogram / manual analysis) were used for analysis. Analyses were performed by Life Science Laboratories Co., Korea. GFP fluorescence linked with lactoferricin peptide measured using a microplate reader (Hidex Sense 425-301) and normalized to the cell density (OD₆₀₀).

### Example 5. Establishment of an integrated biocontainment system

### 5-1. Establishment of an integrated biocontainment system

The strain in which a Q-codon was inserted into the essential gene *cysS* generated in the Example 3-3 was transformed with a plasmid containing the GFP with the Q-codon inserted and the replication initiation gene *trfA* with the Q-codon inserted (To control QtRNA expression at the strain construction stage, a plasmid designed to express QtRNAw under an anhydrotetracycline-inducible promoter was transformed in advance). The number of cases in which the Q-codon was not present in each gene *(cysS, trfA, gfp)* was also produced (8 types in total). The operation of the integrated biocontainment system was confirmed by measuring fluorescence and growth of the completed strain in the same manner as in Examples 4-1 and 4-7.

### Example 6. Establishment of an integrated biocontainment system in Corynebacterium glutamicum

### 6-1. C. Strain and plasmid transformation for C. glutamicum

*Corynebacterium glutamicum* (ATCC21851) was obtained from Korambiotech, Korea. Q-codon inserted GFP gene with H36 promoter was cloned into *E. coli* / *C. glutamicum* shuttle vector pCES208 derivative, and pCES vector was transformed into C. *glutamicum* for fluorescence analysis.

### 6-2. GFP fluorescence measurement for C. glutamicum

Strains for QGFP expression were grown overnight in 2-mL BHI (Brain Heart Infusion) medium supplemented with kanamycin at 37°C, shaking at 200 r.p.m. Cells were diluted to OD₆₀₀ of 0.05 with a fresh BHI media containing kanamycin. Cells were grown at 30°C, shaking at 200 r.p.m. After 24 hours, cells were diluted 1:1000 with PBS, and GFP fluorescence was measured using flow cytometry (Cytoflex S, Beckman Coulter).

### Results

### 1. Arginine Incorporation by E. coli Native Translation System at Quadruplet codon (AGGA) site

To incorporate a canonical amino acid for quadruplet codon (Q-codon), we sought to find a pair of tRNA and aminoacyl-tRNA synthetase derived from the E. coli native translation system. Any Q-codon includes unavoidably one of the existing triplet codons. To avoid unintended cross-reaction, we focused on rare codons of the *E. coli,* and chose the two rarest codons, AGG and AGA encoding arginine, and the cognate tRNAs as the engineering targets (FIG. 1a). We replaced anticodons of the two cognate tRNAs tRNA (tRNA^{Arg} _{CCU} and tRNA^{Arg} ucu) with UCCU, resulting in the Q-tRNAw and Q-tRNAu, respectively. To test quadruplet codon decoding by the Q-tRNAs, we used a green fluorescent protein (GFP) as a reporter. We substituted its original triplet codon for 31R with the Q-codon, AGGA, and also introduced a silent mutation at 45L site, from CGT to CTT, intending to cause a premature termination of translation in case of incorrect Q-codon decoding (FIG. 1b). We obtained four types of strains expressing: 1) GFP with mutation at 45L (positive control), 2) GFP with mutations at 31R and 45L (Q-GFP) (negative control), 3) Q-GFP and Q-tRNAw (W), and 4) Q-GFP and Q-tRNAu (U).

With both Q-tRNAs, the Q-GFP was successfully expressed, which was confirmed by fluorescent measurement (FIG. 1c) and SDS-PAGE (FIG. 1d), whereas either fluorescence (FIG. 1c) or full-length translation (FIG. 1d) was not detected without the Q-tRNAs in the negative control strain. Between the two Q-tRNAs, the Q-tRNAu showed a higher decoding efficiency than the Q-tRNAw. The incorporation of arginine at the Q-codon site was confirmed by MALDI-TOF analysis (FIGs. 1e-g). These results demonstrated that the Q-tRNAs were correctly charged with arginine and decoded the Q-codon in the Q-GFP.

Meanwhile, in order to expand the Q-codon system using AGGA to additional other codons, it was investigated whether Q-GFP is expressed even when the original triplet codon for 31R of green fluorescent protein (GFP) is replaced with the Q-codons AGCA and AGTA, respectively. For this purpose, Q-tRNA was used in which the anticodon of tRNA^{Arg} ecu was replaced with UGCU and UACU, respectively. As a result of checking the expression level of Q-GFP depending on the presence or absence of Q-tRNA expression, it was confirmed that fluorescence was expressed only in the presence of appropriate Q-tRNA even when a gene expression system was constructed using AGCA or AGTA as Q-codons. (FIG. 2).

In subsequent experiments, experiments were performed using AGGA as a Q-codon.

### 2. Engineering Strategies to Improve Quadruplet Codon Decoding

Compared to the GFP expression without the Q-codon, the Q-GFP expression was lower with both Q-tRNAs (FIG. 1c), demanding additional engineering efforts to improve the Q-codon decoding. We supposed that the first three nucleotides (AGG) of the Q-codon (AGGA) were still occupied by the native tRNAs rather than the Q-tRNAs. Therefore, we hypothesized that increasing a pool of the arginine-charged Q-tRNAs or decreasing the competitive native tRNAs will further improve the Q-codon decoding by the Q-tRNAs. To this end, we employed three engineering strategies to: 1) increase the Q-tRNA expression, 2) increase the ArgRS expression, or 3) delete the native tRNA for arginine charging.

To increase the expression level of Q-tRNAs or ArgRS, we constructed additional high-copy plasmids containing each expression cassette for Q-tRNAs or ArgRS, and introduced them into the previously constructed strains. We observed that a boost in the Q-tRNA expression facilitated a higher Q-GFP expression, whereas increasing ArgRS expression did not (FIGs. 3a and 3b).

Next, to decrease the competition between the native tRNAs and the Q-tRNAs, we deleted *argW* gene that encodes tRNA^{Arg} ecu by Lambda Red-mediated recombination, resulting in the *ΔargW* strain. The tRNA ^{Arg} ecu incorporates arginine exclusively in response to the AGG codon, but the AGG codon can be decoded by another tRNA, tRNA^{Arg} _{UCU}. Thus, the absence of tRNA^{Arg} _{UCU} might exert less impact on cellular fitness or proteome. Notably, *ΔargW* strains showed a significant increase in the Q-codon decoding with both Q-tRNAs (FIGs. 3a and 3b), revealing the competition between the Q-tRNA and the native tRNA had restricted the efficient Q-codon decoding. Although the gene deletion increased GFP expression of the positive control that contained no Q-codon, the expression of the Q-tRNAs in the *ΔargW* strain allowed a comparable full-length translation of Q-GFP to the positive control in the wild type strain.

Since *ΔargW* strain did not contain the native tRNA^{Arg} _{CCU}, we were able to confirm aminoacylation status of the Q-tRNAw using a recently developed approach, tRNA Extension (tREX), which consists of oxidation, hybridization, and extension of tRNAs. Briefly, a non-aminoacylated tRNA is subjected to oxidation, but an aminoacylated tRNA is protected from oxidation, enabling the following extension. Thus, a fluorescently labeled DNA-tRNA hybrid can be selectively extended according to the aminoacylation status of the tRNA, and the extension can be visualized in a PAGE gel. After extracting tRNAs from *ΔargW* strain expressing the Q-tRNAw, we performed the tREX protocol with and without the oxidation process, and observed that bands in the same length appeared in the PAGE gel regardless of the oxidation (lane 2 and 4). We also performed the tREX protocol with the same extract but after treating with NAOH to hydrolyse ester bonds between amino acids and tRNAs including the Q-tRNAw. The hydrolysis forced oxidation of the Q-tRNAw, and resulted in a shorter band (lane 3) compared to the previous bands (FIG. 3c). These results suggested aminoacylation of the QtRNAw in the *ΔargW* strain.

### 3. Genome engineering to minimize off-target decoding by the Q-tRNAu

In contrast with the Q-tRNAw, the expression of the Q-tRNAu in the *ΔargW* strain became seriously detrimental to cell growth (FIG. 4a). We hypothesized that a high efficiency of the Q-tRNAu might cause unintended frameshifts in other genes that we were not targeting, possibly resulting in mistranslation of some essential genes. In fact, some essential genes contain the AGG codon for arginine, and the very next base happens to be A, generating AGGA inside their protein coding sequences (CDS), thus the four-base can be misread as the Q-codon by the Q-tRNAs (FIG. 4a). Previous study revealed that 123 arginine codons in essential genes of E. coli are AGG/AGA, and we found that 14 of them contain the AGGA inside their CDSs (FIG. 4b). Thus, we aimed to replace the 14 AGGAs by AGAAs, so as to avoid a potential off-target decoding, but not to alter their amino acids sequence.

To efficiently edit the genome, we combined two genome engineering methods, coselection multiplex automated genome engineering (Cos-MAGE) and CRISPR Optimized MAGE (CRMAGE), resulting in the Cos-CRMAGE (FIG. 4c). Briefly, since we targeted multiple sites on the genome for mutations, we sorted them based on their proximity on the genome, which resulted in the four zones (FIG. 4b). MAGE oligonucleotides were prepared to contain AGAA instead of the original sequence AGGA, and the oligonucleotides of the same zone were applied together during the MAGE cycles to increase mutation efficiency in a cooperative manner. Instead of using antibiotics resistance as a selectable mutation marker, we used a CRISPR/Cas9-based negative selection. We supposed that when no desirable mutations occur on our target sites, which contain a PAM motif (NGG) for Cas9, the Cas9 will create double strand break (DSB) on the genome and kill the cells. We designed single guide RNAs for each target, AGG of the AGGA, to be recognized as the PAM motif, and employed them in accordance with the corresponding group of oligos during the Cos-CRMAGE cycles. For each zone, the Cos-CRMAGE cycle was repeated two to four times at first, and after one series of these cycles, cells were spread onto agar plates for screening. Mutated colonies were screened by multiplex allele-specific colony PCR (MASC-PCR), and the one with the maximum mutations was used as a next starter cell for further mutations. This procedure was repeated until we obtained the final mutant strain with all the 14 mutations (MUT) (FIG. 4d).

We introduced the Q-tRNAu into the mutant strain, and observed that cell-growth was recovered to some extent (FIG. 4e), confirming that minimizing a potential off-target decoding by Q-tRNAs can be beneficial to cell growth. For further experiments, we used the Q-tRNAw for the wild type (WT) or WT *ΔargW* strain (WT *ΔargW*) and the Q-tRNAu for the mutant *ΔargW* strain (MUT *ΔargW*)*.*

### 4. Quadruplet Codon Decoding System as a Genetic Biocontainment Means

Having demonstrated the efficient Q-codon decoding by the native translation system-derived approach, we sought to confirm whether the Q-codon decoding system can be used as a genetic biocontainment means to protect: 1) genetic traits, 2) genetic materials, 3) engineered strains, and 4) genetic information.

### 4-1. Q-codon Decoding System to confine genetic traits in a permissive environment

We assumed that the Q-codon incorporation into a target gene or metabolic pathway would prevent unintended gene expression or metabolite synthesis in the non-permissive environment lacking the Q-codon decoding system. To demonstrate this, we first applied the Q-codon decoding system to regulate a gene expression for antibiotics resistance using the Q-tRNAw and WT *ΔargW* strain. We introduced the Q-codon into the gene for kanamycin resistance. We observed that the cells containing the Q-tRNAw were only able to survive in response to the kanamycin (FIG. 5a).

We further applied the Q-codon decoding system as a genetic regulatory means for a heterologous metabolic pathway. We placed the Q-tRNAu gene under the arabinose-inducible promoter, PBAD, aiming to regulate expression of the Q-tRNA. As a target metabolic pathway, we chose lycopene production pathway, which consist of three enzymes: geranylgeranyl diphosphate synthase (CrtE), phytoene synthase (CrtB), and phytoene desaturase (CrtI) (FIG. 5b). We introduced the Q-codon into three enzymes and examined lycopene production depending on the Q-tRNAu expression in the MUT *ΔargW* strain. Notably, we observed that lycopene was produced only when the Q-tRNA was co-expressed (FIGs. 5c, 5d). Together, these results showed that genes or pathways can be regulated by the Q-codon decoding system, suggesting its utility to confine genetic traits in a permissive environment.

### 4-2. Q-codon Decoding Svstem as a Preventive against the Leakage of genetic material

Genetic material (e.g. plasmid) can be transferred to the environment or other microorganism, causing a potential environmental or health risk. We aimed to develop a preventive measure against the leakage of genetic material to a non-permissive environment using the Q-codon decoding system.

The pBAC/oriV plasmids, which contain two replication origins, can switch the copy number from single to multi. TrfA, which is a replication initiation protein, must bind to the oriV, turning on its multi-copy replication state. Based on this replication mechanism, we hypothesized that if a plasmid contains the oriV as its only replication origin and TrfA contains the Q-codon in the CDS, the plasmid cannot be maintained in a non-permissive environment where there is no Q-codon decoding system (FIG. 6a). To test this hypothesis, we synthesized the trfA gene containing the Q-codon at 156R site (Q-trfA) under a constitutive promoter, J23110, and placed the resulting expression cassette downstream of QGFP in the pQGFP. The original ClodF13 origin of replication was replaced by the oriV derived from pBAC/oriV, yielding pQtrfA_oriV. After co-transformation of pQtrfA_oriV and pH.Badw to WT *ΔargW* strain, cells were spread onto two separate LB plates containing kanamycin with and without arabinose. Colonies appeared only on the arabinose-containing plate, indicating that the plasmid containing the oriV was not able to be maintained in a cell when no Q-codon decoding system was activated (FIGs. 6b, 6c). The result suggested that we can restrict the proliferation of genetic material in a non-permissive environment by making its replication to be dependent on the Q-codon decoding system.

### 4-3. Q-codon Decoding System as a Preventive against the Leakage of engineered strains

Next, we sought to investigate whether the Q-codon decoding system can be utilized as a preventive measure against the leakage of engineered strains. We hypothesized that inserting the Q-codon to an essential enzyme, which is responsible for cell growth, would increase the cell's dependency on the Q-codon decoding, preventing unintended proliferation in a non-permissive environment (FIG. 7a). We chose two enzymes (cysS: cysteine-tRNA ligase, fldA: flavodoxin 1) that are essential for cell growth and introduced AGGA into one of the arginine sites of each gene on the genome. We employed the CRMAGE for genome engineering. Oligonucleotide, which contains the target mutation, AGGA, and another silent mutation for negative selection, was transformed to WT *ΔargW* strain expressing the Q-tRNAw under a constitutive promoter. After confirming the mutation, we replaced the Q-tRNAw expression plasmid by the other plasmid expressing the Q-tRNAw under an ATc inducible promoter, P_{Tet}. We observed that cells were able to survive only when the Q-tRNAs were expressed by ATc induction, or grow very slowly without the Q-tRNAs (FIGs. 7b-c).

### 4-4. Q-codon Decoding System for sequence encryption

In addition to biological substances like genetic material or strains, genetic information must be protected from leakage or theft; thus it necessitates development of another protection layer. We sought to exploit "intentional frameshifts" by the insertion of Q-codon as a means for sequence encryption.

We selected lactoferricin derivative, which is a relatively short antimicrobial peptide (AMP) and contains arginine near its N-terminal. We compared their sequences of amino acids and nucleotides with and without the Q-codon using BLAST tools. Not surprisingly, the insertion of Q-codon sufficiently altered amino acids downstream, hiding its identity in BLASTP. However, in BLASTN, the Q-codon was not solely able to hide its identity; thus we altered one nucleotide additionally, which did not change the amino acid, but resulted in effective concealment (Table 9).

**[Table 9]**

| | | | |
|---|---|---|---|
| Q-codon | - | + | + |
| Additional Silent mutation | - | - | + |
| Amino acids sequence | FKCWRWQWRWK KLGA | FKCWRMAVALEK TGC | FKCWRMAMALE KTGC |
| Nucleotide sequence | TTCAAATGCTGGC | TTCAAATGCTGGA | TTCAAATGCTGGA |
| | GTTGGCAGTGGC GTTGGAAAAAAC TGGGTGCT | GGATGGCAGTGG CGTTGGAAAAAA CTGGGTGCT | GGATGGCAATGG CGTTGGAAAAAA CTGGGTGCT |

| BLAST SEARCH | | | |
|---|---|---|---|
| BLASTP | Detected | **Not detected** | **Not detected** |
| BLASTN | Detected | Detected | **Not detected** |

In addition, it was confirmed by fluorescence through fusion with GFP that the lactoferricin with the Q-codon inserted was actually expressed in the permissive environment (FIG. 8b), and it was confirmed by MS analysis that the actually expressed Q-lactoferricin peptide sequence was identical to the lactoferricin without Q-codon (FIGs. 8c, 8d).

### 5. Establishment of an integrated biocontainment system using Q-codon

Through the above experiments, it was confirmed that it was possible to establish a biocontainment system by inserting the Q-codon into the target protein, essential gene, or plasmid replication initiation gene. Since it was confirmed that each strategy of inserting a Q-codon into each gene works well, in order to construct a biocontainment system that integrates these strategies, we inserted Q-codon in each of the target protein, plasmid replication initiation gene, and essential gene within one strain. Further, in order to confirm the growth and protein expression levels according to the combination of Q-codon presence and absence, we made all possible combinations of Q-codon presence and absence within one strain. There were a total of 8 types of combinations, ranging from (0, 0, 0), which means no Q-codon in three genes (i.e., essential gene, plasmid replication initiation gene, and target protein), to (1, 1, 1), which means all three genes have Q-codon. 0 in parentheses means that the Q codon does not exist, and 1 means that the Q codon exists. The genes corresponding to each position represent *cysS, trfA,* and *gfp* in that order. Thus, for example, (1, 0, 0) means Q-codon in cysS (essential gene), (0, 1, 0) means Q-codon in trfA (plasmid replication initiation gene), and (0, 1, 1) means Q-codon in GFP (target protein).

As a result, as shown in FIG. 9, strains with Q-codon in *cysS* {(1, 0, 0), (1, 0, 1), (1, 1, 0), (1, 1, 1)} were able to survive only when the Q-tRNA was expressed. When Q-tRNA was not expressed, the strain did not show growth. Even if there is no Q-codon in the *cysS,* strains with Q-codon in *trfA* {(0, 1, 0) and (0, 1, 1)} were also able to survive only when the Q-tRNA was expressed because the antibiotic resistance plasmid was maintained. When Q-tRNA was not expressed, it lost antibiotic resistance and did not show growth. Strains with no Q-codon in both cysS and trfA {(0, 0, 0) and (0, 0, 1)} can survive regardless of whether Q-tRNA is expressed or not, but strain with Q-codon in the GFP {(0, 0, 1)} were able to express GFP only when the Q-tRNA was expressed. In the case of the wild type {(0, 0, 0)}, which lacks Q-codons in all three genes, strains always survived and protein was always expressed.

### 6. Q-codon expression in Corynebacterium glutamicum

After constructing the Q-codon system in *E. coli,* the Q-codon system was applied to *Corynebacterium Glutamicum* to confirm whether the present invention can be applied to other strains. We replaced anticodon of the Arginine tRNA_CCU in *Corynebacterium Glutamicum* with UCCU to create Coryne_QtRNA_UCCU. To test quadruplet codon decoding by the Q-tRNA in Corynebacterium, we used green fluorescence protein (GFP) as a reporter. We substituted its original triplet codon for 31R with the Q-codon, AGGA. *E. coli* / *C. glutamicum* shuttle vector pCES208 was used for expression in *C*. *glutamicum.* As shown in FIG. 10, Q-GFP was successfully expressed with Q-tRNA expression in C. *glutamicum.* Fluorescence was measured by flowcytometry.

Based on the above description, it will be understood by those skilled in the art that the present disclosure may be implemented in a different specific form without changing the technical spirit or essential characteristics thereof. In this regard, it should be understood that the above embodiment is not limitative, but illustrative in all aspects. The scope of the disclosure is defined by the appended claims rather than by the description preceding them, and therefore all changes and modifications that fall within metes and bounds of the claims, or equivalents of such metes and bounds, are intended to be embraced by the claims.

## Claims

1. A genetic biocontainment system comprising a nucleic acid molecule in which a triplet codon encoding a specific amino acid in a nucleic acid sequence has been replaced with a quadruplet codon,
wherein the nucleic acid molecule is expressed as a protein in the presence of (i) a tRNA with an anticodon complementary to the quadruplet codon and (ii) an aminoacyl-tRNA synthetase (AARS) that recognizes and acylates the tRNA, and the nucleic acid molecule is not expressed as a protein in the absence of the tRNA with an anticodon complementary to the quadruplet codon.

2. The system of claim 1, wherein the nucleic acid molecule is one, two or three types of nucleic acid molecules selected from the group consisting of a gene encoding a target protein, a gene encoding a plasmid replication initiation protein, and a gene encoding a protein essential for cell growth.

3. The system of claim 1 or 2, wherein the nucleic acid molecule is a gene encoding a target protein, and the nucleic acid molecule is not expressed as the target protein in the absence of the tRNA with an anticodon complementary to the quadruplet; or
wherein the nucleic acid molecule is a gene involved in a metabolite synthesis pathway, and metabolites are not synthesized in the absence of the tRNA with an anticodon complementary to the quadruplet; or
wherein the nucleic acid molecule is a gene encoding a plasmid replication initiation protein, and the plasmid containing the nucleic acid molecule does not replicate in the absence of the tRNA with an anticodon complementary to the quadruplet; or
wherein the nucleic acid molecule is a gene encoding a protein essential for cell growth, and cell death does not occur in the absence of the tRNA with an anticodon complementary to the quadruplet.

4. The system of any one of claims 1 to 3, wherein the nucleic acid molecule is a nucleic acid molecule in which a triplet codon encoding arginine in a nucleic acid sequence has been replaced with a quadruplet codon.

5. The system of claim 4, wherein the nucleic acid molecule is a nucleic acid molecule in which a triplet codon encoding arginine in a nucleic acid sequence has been replaced with a quadruplet codon consisting of AGNN (N is A, T, C or G).

6. A microbial strain for use in genetic biocontainment, comprising a nucleic acid molecule in which a triplet codon encoding a specific amino acid in a nucleic acid sequence has been replaced with a quadruplet codon,
wherein the microbial strain comprises a quadruplet codon decoding system comprising (i) a tRNA with an anticodon complementary to the quadruplet codon and (ii) an aminoacyl-tRNA synthetase (AARS) that recognizes and acylates the tRNA, and
wherein the expression of the tRNA is controlled under an inducible promoter, such that in the presence of an inducer of the inducible promoter, the tRNA is expressed and the nucleic acid molecule is expressed as a protein, and in the absence of the inducer, the tRNA is not expressed and the nucleic acid molecule is not expressed as a protein.

7. The microbial strain of claim 6, wherein the nucleic acid molecule is one, two or three types of nucleic acid molecules selected from the group consisting of a gene encoding a target protein, a gene encoding a plasmid replication initiation protein, and a gene encoding a protein essential for cell growth.

8. The microbial strain of claim 6 or 7, wherein the nucleic acid molecule is a gene encoding a target protein, and the nucleic acid molecule is not expressed as the target protein in the absence of the inducer of the inducible promoter for the tRNA is absent; or
wherein the nucleic acid molecule is a gene involved in a metabolite synthesis pathway, and metabolites are not synthesized in the absence of the inducer of the inducible promoter for the tRNA is absent; or
wherein the nucleic acid molecule is a gene encoding a plasmid replication initiation protein, and the plasmid containing the nucleic acid molecule does not replicate in the absence of the inducer of the inducible promoter for the tRNA is absent; or
wherein the nucleic acid molecule is a gene encoding a protein essential for cell growth, and the microbial strain dies in the absence of the inducer of the inducible promoter for the tRNA is absent.

9. The microbial strain of any one of claims 6 to 8, wherein (i) the tRNA with an anticodon complementary to the quadruplet codon and (ii) the aminoacyl-tRNA synthetase (AARS) that recognizes and acylates the tRNA are a modified version of the endogenous tRNA/AARS system of the microbial strain.

10. The microbial strain of any one of claims 6 to 9, wherein the nucleic acid molecule is a nucleic acid molecule in which a triplet codon encoding arginine in a nucleic acid sequence has been replaced with a quadruplet codon.

11. The microbial strain of claim 10, wherein the nucleic acid molecule is a nucleic acid molecule in which a triplet codon encoding arginine in a nucleic acid sequence has been replaced with a quadruplet codon consisting of AGNN (N is A, T, C or G).

12. The microbial strain of claim 10 or 11, wherein the tRNA with an anticodon complementary to the quadruplet codon is a tRNA produced by replacing the anticodon of the strain's endogenous tRNA for arginine with the anticodon complementary to the quadruplet codon.

13. The microbial strain of any one of claims 10 to 12, wherein the microbial strain is a microbial strain in which argW gene encoding the strain's endogenous tRNA for arginine has been knocked out.

14. A genetic biocontainment method comprising:
providing the system of any of claims 1 to 5 or the microbial strain of any of claims 6 to 13; and
inducing or blocking the expression of a nucleic acid molecule containing the quadruplet codon depending on the presence or expression of a tRNA with an anticodon complementary to the quadruplet codon.

15. A biocontainment method for sequence information comprising:
replacing a triplet codon encoding a specific amino acid in a nucleic acid sequence with a quadruplet codon to cause a frameshift.
